# EUROPEAN PATENT APPLICATION

(11) **EP 4 589 336 A1**
(43) Date of publication of application: **23.07.2025**
(21) Application number: 23865574.0
(22) Date of filing: 13.09.2023
(51) Int. Cl.: G01S 13/34, A61B 5/0245, A61B 5/11, G01S 13/88

(54) **ELECTRONIC DEVICE, METHOD FOR CONTROLLING ELECTRONIC DEVICE, AND PROGRAM**

(30) Priority: 16.09.2022 JP 2022148632
(71) Applicant: Kyocera Corporation, Kyoto-shi, Kyoto 612-8501 (JP)
(72) Inventor: KURODA Jun, Kyoto-shi, Kyoto 612-8501 (JP); MURAKAMI Youhei, Kyoto-shi, Kyoto 612-8501 (JP); SAHARA Tooru, Kyoto-shi, Kyoto 612-8501 (JP); HOMMA Takuya, Kyoto-shi, Kyoto 612-8501 (JP); KASHIMA Yuu, Kyoto-shi, Kyoto 612-8501 (JP); TAKAMATSU Tetsuya, Kyoto-shi, Kyoto 612-8501 (JP)
(74) Representative: Viering, Jentschura & Partner mbB Patent- und Rechtsanwälte
(86) International application number: PCT/JP2023/033444
(87) International publication number: WO 2024/058225

(57) **Abstract**

An electronic device includes a transmission unit, a reception unit, and a signal processing unit. The transmission unit is configured to transmit a transmission wave. The reception unit is configured to receive a reflected wave of the transmission wave from a target. The signal processing unit is configured to detect a distance position, direction, and velocity of the target on the basis of a converted signal obtained by taking the Fourier transform of a beat signal of the transmission wave and the reflected wave. The signal processing unit includes an extraction unit. The extraction unit is configured to extract heartbeat information about the target on the basis of the detected distance, direction, and velocity of the target. The extraction unit is configured to extract signal components corresponding to vibration associated with the heartbeat of the target from the converted signal by using a plurality of window functions. The extraction unit is configured to select heart sound signals that are most appropriate as heart sound by applying a learning classifier to the extracted signal components. The extraction unit is configured to select an optimum signal that is most appropriate as the heartbeat by performing envelope processing on the most appropriate heart sound signals. The extraction unit is configured to acquire values indicating intervals between peak times from the optimum signal, and extract a heartbeat interval of the target on the basis of the variance and the median of the values indicating intervals.

## Description

### CROSS-REFERENCE TO RELATED APPLICATIONS

This application claims priority based on Japanese Patent Application No. 2022-148632 filed September 16, 2022, the entire disclosure of which is hereby incorporated by reference.

### TECHNICAL FIELD

The present disclosure relates to an electronic device, a method of controlling an electronic device, and a program.

### BACKGROUND OF INVENTION

In fields such as the automotive and related industries, for example, technologies for measuring values such as the distance between a vehicle and a given object are gaining importance. In particular, recent years have seen various research into radio detection and ranging (RADAR) technology, which measures values such as the distance to an obstacle or other object by transmitting radio waves, such as millimeter waves, and receiving reflected waves back from the object. The importance of technologies for measuring such distances and the like is expected to increase further with the development of technologies for assisting drivers with driving and technologies related to self-driving, in which driving is partially or fully automated.

Various technologies have been proposed to detect the presence or the like of a given object by receiving reflected waves of transmitted radio waves reflecting off the object. For example, Patent Literature 1 proposes a device that can detect the presence of a person and biological information about the person by using microwaves. As another example, Patent Literature 2 proposes a device that detects vital signs such as the respiration or heartbeat frequency of a living body on the basis of reflected microwave radar signals.

### CITATION LIST

### PATENT LITERATURE

Patent Literature 1: Japanese Unexamined Patent Application Publication No. 2002-71825
Patent Literature 2: Japanese Unexamined Patent Application Publication No. 2021-32880

### SUMMARY

An embodiment of the present disclosure is an electronic device. The electronic device includes a transmission unit, a reception unit, and a signal processing unit. The transmission unit is configured to transmit a transmission wave. The reception unit is configured to receive a reflected wave of the transmission wave from a target. The signal processing unit is configured to detect a distance position, direction, and velocity of the target on the basis of a converted signal obtained by taking the Fourier transform of a beat signal of the transmission wave and the reflected wave. The signal processing unit includes an extraction unit. The extraction unit is configured to extract heartbeat information about the target on the basis of the detected distance, direction, and velocity of the target. The extraction unit is configured to extract signal components corresponding to vibration associated with the heartbeat of the target from the converted signal by using a plurality of window functions. The extraction unit is configured to select heart sound signals that are most appropriate as heart sound by applying a learning classifier to the extracted signal components. The extraction unit is configured to select an optimum signal that is most appropriate as the heartbeat by performing envelope processing on the most appropriate heart sound signals. The extraction unit is configured to acquire values indicating intervals between peak times from the optimum signal, and extract a heartbeat interval of the target on the basis of the variance and the median of the values indicating intervals.

An embodiment of the present disclosure is a method of controlling an electronic device. The method involves transmitting a transmission wave from a transmission unit. The method involves receiving, at a reception unit, a reflected wave of the transmission wave from a target. The method involves detecting a distance position, direction, and velocity of the target on the basis of a converted signal obtained by taking the Fourier transform of a beat signal of the transmission wave and the reflected wave. The method involves extracting heartbeat information about the target on the basis of the detected distance, direction, and velocity of the target. The extracting of heartbeat information about the target involves extracting signal components corresponding to vibration associated with the heartbeat of the target from the converted signal by using a plurality of window functions. The extracting of heartbeat information about the target involves selecting heart sound signals that are most appropriate as heart sound by applying a learning classifier to the extracted signal components. The extracting of heartbeat information about the target involves selecting an optimum signal that is most appropriate as the heartbeat by performing envelope processing on the most appropriate heart sound signals. The extracting of heartbeat information about the target involves acquiring values indicating intervals between peak times from the optimum signal, and extracting a heartbeat interval of the target on the basis of the variance and the median of the values indicating intervals.

An embodiment of the present disclosure is a program. The program causes an electronic device to perform a process. The process involves transmitting a transmission wave from a transmission unit. The process involves receiving, at a reception unit, a reflected wave of the transmission wave from a target. The process involves detecting a distance position, direction, and velocity of the target on the basis of a converted signal obtained by taking the Fourier transform of a beat signal of the transmission wave and the reflected wave. The process involves extracting heartbeat information about the target on the basis of the detected distance, direction, and velocity of the target. The extracting of heartbeat information about the target involves extracting signal components corresponding to vibration associated with the heartbeat of the target from the converted signal by using a plurality of window functions. The extracting of heartbeat information about the target involves selecting heart sound signals that are most appropriate as heart sound by applying a learning classifier to the extracted signal components. The extracting of heartbeat information about the target involves selecting an optimum signal that is most appropriate as the heartbeat by performing envelope processing on the most appropriate heart sound signals. The extracting of heartbeat information about the target involves acquiring values indicating intervals between peak times from the optimum signal, and extracting a heartbeat interval of the target on the basis of the variance and the median of the values indicating intervals.

### BRIEF DESCRIPTION OF THE DRAWINGS

FIG. 1 is a diagram for describing how an electronic device according to an embodiment is used.
FIG. 2 is a function block diagram schematically illustrating a configuration of an electronic device according to an embodiment.
FIG. 3 is a diagram for describing the composition of a signal processed by an electronic device according to an embodiment.
FIG. 4 is a diagram for describing the processing of a signal by an electronic device according to an embodiment.
FIG. 5 is a diagram for describing the processing of a signal by an electronic device according to an embodiment.
FIG. 6 is a diagram for describing the processing of a signal by an electronic device according to an embodiment.
FIG. 7 schematically illustrates an example of the antenna arrangement and the operating principle of an antenna array of an electronic device according to an embodiment.
FIG. 8 illustrates an example of the antenna arrangement in an antenna array of an electronic device according to an embodiment.
FIG. 9 illustrates an example of the processing of a signal by an electronic device according to an embodiment.
FIG. 10 illustrates an example of the processing of a signal by an electronic device according to an embodiment.
FIG. 11 is a flowchart for describing a comparative example of operations by an electronic device according to an embodiment.
FIG. 12 is a flowchart for describing operations by an electronic device according to an embodiment.
FIG. 13 is a flowchart for describing operations by an electronic device according to an embodiment.
FIG. 14 is a diagram for describing multi-window processing by an electronic device according to an embodiment.
FIG. 15 illustrates the relationship between ranks representing an objective signal and a noise signal in singular value decomposition by an electronic device according to an embodiment.
FIG. 16 conceptually illustrates multiresolution analysis using a discrete wavelet transform by an electronic device according to an embodiment.
FIG. 17 is a diagram for conceptually describing processing to classify and discriminate the best heart sound envelope curve by an electronic device according to an embodiment.
FIG. 18 illustrates the result of performing one-dimensionalization processing using the continuous wavelet transform in an electronic device according to an embodiment.
FIG. 19 conceptually illustrates machine learning by an electronic device according to an embodiment.
FIG. 20 illustrates an example of the classification and discrimination of a signal by an electronic device according to an embodiment.
FIG. 21 illustrates an example of the mean absolute percent error of the best heart sound envelope curve by an electronic device according to an embodiment.
FIG. 22 illustrates an enlarged view of two heart sound envelopes detected by an electronic device according to an embodiment.
FIG. 23 illustrates a histogram of heart sound peak-to-peak intervals detected by an electronic device according to an embodiment.
FIG. 24 is a diagram for describing the extraction of heart sound envelope curves by an electronic device according to an embodiment.
FIG. 25 is a diagram for describing DP matching for RRI estimation by an electronic device according to an embodiment.
FIG. 26 illustrates a heart sound time-series waveform obtained by an electronic device according to an embodiment.
FIG. 27 illustrates a heart sound time-series waveform obtained by an electronic device according to an embodiment.
FIG. 28 illustrates an RRI time-series waveform obtained by an electronic device according to an embodiment.
FIG. 29 illustrates an RRI power spectral density obtained by an electronic device according to an embodiment.
FIG. 30 illustrates an example of the antenna arrangement in an antenna array of an electronic device according to an embodiment.
FIG. 31 is a diagram for describing negative peaks in the reconstruction error output of an autoencoder in processing by an electronic device according to an embodiment.
FIG. 32 is a diagram for describing negative peaks in the reconstruction error output of an autoencoder in processing by an electronic device according to an embodiment.

### DESCRIPTION OF EMBODIMENTS

The ability to detect weak vibrations, such as the heartbeat of a human body, with good precision by transmitting and receiving radio waves, such as millimeter waves for example, could be useful in a wide variety of fields. An objective of the present disclosure is to provide an electronic device, a method of controlling an electronic device, and a program with which the heartbeat of a human body or the like can be detected with good precision by transmitting and receiving radio waves. According to an embodiment, it is possible to provide an electronic device, a method of controlling an electronic device, and a program with which the heartbeat of a human body or the like can be detected with good precision by transmitting and receiving radio waves. The following describes an embodiment in detail, with reference to the drawings.

In the present disclosure, an "electronic device" may be a device driven by electric power. A "user" may be an entity (typically a human being) or an animal that uses a system and/or an electronic device according to an embodiment. The user may include an entity that monitors a human being or other subject by using an electronic device according to an embodiment. The "subject" may be an entity (a human being or an animal, for example) to be monitored using an electronic device according to an embodiment. The user may also include the subject.

In the present disclosure, "heartbeat" may refer to the pulsation of the heart. "Pulsation" may refer to a rhythmic contraction movement performed by the heart.

In the present disclosure, "heart rate" refers to the number of times the heart pulsates in a certain period of time. For example, the heart rate may refer to the number of pulsations per minute. Pulsations occur in the arteries when the heart pumps blood. Accordingly, the number of pulsations by the arteries may also be referred to as the pulse rate or simply the pulse.

In the present disclosure, "heart sound" may refer to the sound of a beating heart. That is, the heart sound may refer to the sound that occurs when the heart contracts and dilates. The "heart sound" may consist of a low, long first sound based on ventricular muscle tension, mitral valve closure, initiation of blood ejection into the arteries, and/or acceleration of blood flow, followed by a high, short second sound derived from aortic valve closure and/or pulmonary valve closure.

In the present disclosure, the heart sound is not necessarily limited to physical sounds based on air vibrations, and may also mean the vibrations themselves caused by the beating (pulsation) of the heart. For example, in the present disclosure, the heartbeat may be assumed to imply a vibrating source, and the heart sound may be assumed to imply the vibrations themselves caused by the vibrating source. In the present disclosure, the heartbeat may also be assumed to imply the heart sound, depending on the situation.

In the present disclosure, the heart sound may refer to the vibrations of the body in association with the movement of the heart. For example, the heart sound may refer to vibrations of the entire body or of the head, neck, chest, throat, arm, leg, wrist, or some other part of the body. For example, the heart sound may refer to vibrations of the skin or the like on the surface of the entire body or of the head, neck, chest, throat, arm, leg, wrist, or some other part of the body. For example, the heart sound may refer to vibrations of clothing, underwear, eyeglasses, or other items worn by a test subject in association with vibrations of the skin or the like on the surface of the entire body or of the head, neck, chest, throat, arm, leg, wrist, or some other part of the body. The heartbeat may refer to the pulsations themselves of the heart. A heartbeat interval, heart rate, or the like may be calculated from the movement of the heartbeat. In the present disclosure, the pulsation of the heart also be referred to as beating.

An electronic device according to an embodiment can detect the heartbeat of a human being or other subject present in the surroundings of the electronic device. Accordingly, anticipated situations in which an electronic device according to an embodiment is used may be, for example, specific facilities or the like used by people engaged in social activities, such as companies, hospitals, nursing homes, schools, sports gyms, and nursing care facilities. For example, it is extremely important for a company to grasp and/or manage the health status of its employees and the like. Similarly, it is extremely important for a hospital to grasp and/or manage the health status of its patients, medical personnel, and the like, and for a nursing home to grasp and/or manage the health status of its residents, staff, and the like. The situations in which an electronic device according to an embodiment is used are not limited to facilities such as companies, hospitals, and nursing homes as described above, and may be any given facility where it is desirable to grasp and/or manage the health status of a subject. The any given facility may also include a non-commercial facility, such as the home of a user. The situations in which an electronic device according to an embodiment is used are not limited to indoors and may also be outdoors. For example, the situations in which an electronic device according to an embodiment is used may also be inside means of transportation such as trains, buses, airplanes, and the like, as well as stations, boarding areas, and the like. The situations in which an electronic device according to an embodiment is used may also be a means of transportation such as an automobile, aircraft, or ship, a hotel, the home of a user, or the living room, bath, toilet, bedroom, or the like in a home.

In a nursing facility, for example, an electronic device according to an embodiment may be used for the purpose of detecting or monitoring the heartbeat of a subject such as a person in need of nursing or care. If an abnormality is found in the heartbeat of a subject such as a person in need of nursing or care, for example, an electronic device according to an embodiment may also issue a predetermined warning to the person in question and/or another person. Consequently, according to an electronic device according to an embodiment, the person in question and/or a staff member of a nursing care facility or the like may recognize that an abnormality is found in the pulse of a subject such as a person in need of nursing or care, for example. On the other hand, if an abnormality is not found (for example, the heartbeat is found to be normal) in the heartbeat of a subject such as a person in need of nursing or care, for example, an electronic device according to an embodiment may also issue a notification to that effect to the person in question and/or another person. Consequently, according to an electronic device according to an embodiment, the person in question and/or a staff member of a nursing care facility or the like may recognize that the pulse is normal for a subject such as a person in need of nursing or care, for example.

An electronic device according to an embodiment may also detect the pulse of an animal other than a human being as the subject. As an example, the electronic device according to an embodiment described hereinafter is described as detecting the pulse of a human being using a sensor based on a technology like millimeter-wave radar.

An electronic device according to an embodiment may be installed in any stationary object, and may also be installed in any moving object. An electronic device according to an embodiment can transmit a transmission wave from a transmitting antenna to the surroundings of the electronic device. An electronic device according to an embodiment can receive, from a receiving antenna, a reflected wave resulting from the transmission wave being reflected. At least one of the transmitting antenna or the receiving antenna may be provided in the electronic device, and may also be provided in a radar sensor, for example.

The following describes a typical example in which an electronic device according to an embodiment is stationary. On the other hand, the subject (human being) whose pulse is to be detected by an electronic device according to an embodiment may be stationary, moving, or moving their body while remaining stationary. Like an ordinary radar sensor, an electronic device according to an embodiment can measure the distance and the like between the electronic device and an object in the surroundings of the electronic device in situations in which the object may move. An electronic device according to an embodiment can measure the distance and the like between the electronic device and an object even if the electronic device and the object are both stationary.

The following describes an electronic device according to an embodiment in detail, with reference to the drawings. First, an example of the detection of an object by an electronic device according to an embodiment will be described.

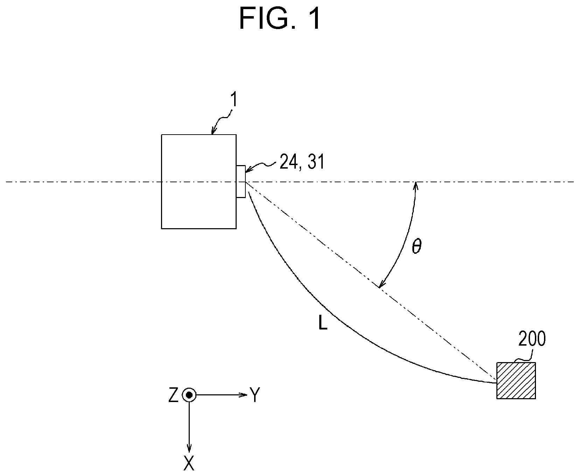

FIG. 1 is a diagram for describing an example of how an electronic device according to an embodiment is used. FIG. 1 illustrates an example of an electronic device provided with the functions of a sensor provided with a transmitting antenna and a receiving antenna according to an embodiment.

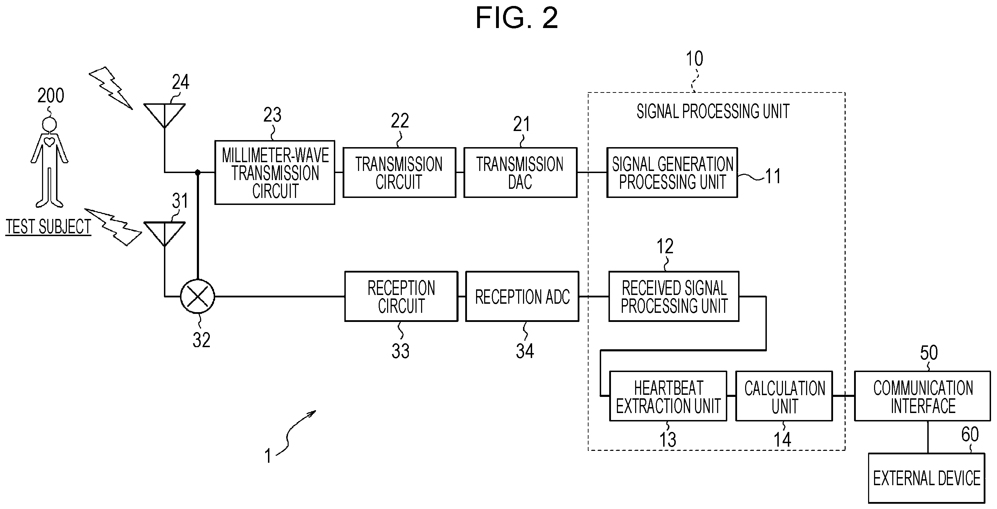

As illustrated in FIG. 1, in an embodiment, an electronic device 1 may be provided with a transmission unit and a reception unit. As described later, the transmission unit may be provided with a transmitting antenna 24. The reception unit may be provided with a receiving antenna 31. Specific configurations of the electronic device 1, the transmission unit, and the reception unit will be described later. For simplicity, FIG. 1 illustrates a situation in which the electronic device 1 is provided with the transmitting antenna 24 and the receiving antenna 31. The electronic device 1 may also include at least one other functional unit, as appropriate, such as at least a portion of a signal processing unit 10 (FIG. 2) included in the electronic device 1. The electronic device 1 may be provided with at least one other functional unit outside the electronic device 1, such as at least a portion of a signal processing unit 10 (FIG. 2) included in the electronic device 1. In FIG. 1, the electronic device 1 may be moving, but may also be stationary without moving.

The example illustrated in FIG. 1 illustrates in a simplified manner the transmission unit provided with the transmitting antenna 24 and the reception unit provided with the receiving antenna 31 in the electronic device 1. The electronic device 1 may also be provided with a plurality of transmission units and/or a plurality of reception units, for example. The transmission unit may be provided with a transmitting antenna 24 formed from a plurality of transmitting antennas. The reception unit may be provided with a receiving antenna 31 formed from a plurality of receiving antennas. The position where the transmission unit and/or reception unit are installed in the electronic device 1 is not limited to the position illustrated in FIG. 1, and may be another position, as appropriate. The number of transmission units and/or reception units may be any number equal to or greater than 1, according to various conditions (or requirements) such as the range and/or precision of heartbeat detection by the electronic device 1.

As described later, the electronic device 1 transmits an electromagnetic wave as a transmission wave from the transmitting antenna 24. For example, if a given object (for example, the subject 200 illustrated in FIG. 1) is present in the surroundings of the electronic device 1, at least a portion of the transmission wave transmitted from the electronic device 1 is reflected by the object to become a reflected wave. Such a reflected wave is then received by the receiving antenna 31 of the electronic device 1, for example, whereby the electronic device 1 can detect the subject as a target.

Typically, the electronic device 1 provided with the transmitting antenna 24 may be a radio detection and ranging (RADAR) sensor that transmits and receives radio waves. However, the electronic device 1 is not limited to a radar sensor. In an embodiment, the electronic device 1 may also be, for example, a sensor based on light detection and ranging (LIDAR) technology, also known as laser imaging detection and ranging, which involves light waves. Sensors such as these can be configured to include a patch antenna or the like. Since technologies such as RADAR and LIDAR are already known, a detailed description may be simplified or omitted, as appropriate. In an embodiment, the electronic device 1 may also be a sensor based on a technology that detects objects by transmitting and receiving sonic or ultrasonic waves, for example.

The electronic device 1 illustrated in FIG. 1 receives from the receiving antenna 31 a reflected wave of a transmission wave transmitted from the transmitting antenna 24. With this arrangement, the electronic device 1 can detect a given subject 200 present within a given distance from the electronic device 1 as a target. For example, as illustrated in FIG. 1, the electronic device 1 can measure the distance L between the electronic device 1 and a given subject 200. The electronic device 1 can also measure the relative velocity between the electronic device 1 and a given subject 200. The electronic device 1 can also measure the direction (angle of arrival θ) from which the reflected wave from a given subject 200 arrives at the electronic device 1.

In FIG. 1, the XY plane may be defined as the plane substantially parallel to the ground, for example. In this case, the positive direction of the Z axis illustrated in FIG. 1 may indicate the vertically upward direction. In FIG. 1, the electronic device 1 may be installed on a plane parallel to the XY plane. In FIG. 1, the subject 200 may be standing on the ground substantially parallel to the XY plane, for example.

The subject 200 may be a human being or the like present in the surroundings of the electronic device 1, for example. The subject 200 may also be a living thing other than a human being, such as an animal, present in the surroundings of the electronic device 1, for example. As described above, the subject 200 may be moving, and may also be stopped or stationary. In the present disclosure, the object to be detected by the electronic device 1 includes inanimate things such as any object, as well as living things such as people, dogs, cats, horses, and other animals. The object to be detected by the electronic device 1 according to the present disclosure may also include a target, including a person, a thing, an animal, or the like, that is detected by radar technology. In the present disclosure, a target may include a person, a thing, an animal, or the like. The following description assumes that the object such as the subject 200 present in the surroundings of the electronic device 1 is a human being (or an animal). Hereinafter, the "subject 200" is also referred to as the "test subject 200", as appropriate. In the present disclosure, the target may also be the subject 200 above.

In FIG. 1, the ratio of the size of the electronic device 1 to the size of the subject 200 does not necessarily indicate the actual ratio. In FIG. 1, the transmitting antenna 24 of the transmission unit and the receiving antenna 31 of the reception unit are illustrated as being installed on the outside of the electronic device 1. However, in an embodiment, the transmitting antenna 24 of the transmission unit and/or the receiving antenna 31 of the reception unit may be installed at various positions in the electronic device 1. For example, in an embodiment, the transmitting antenna 24 of the transmission unit and/or the receiving antenna 31 of the reception unit may be installed inside the electronic device 1, and may not appear on the exterior of the electronic device 1.

The following describes a typical example in which the transmitting antenna of the electronic device 1 transmits radio waves in a frequency band such as millimeter waves (30 GHz or higher) or quasi-millimeter waves (for example, around 20-30 GHz). On the other hand, the transmitting antenna of the electronic device 1 may also transmit radio waves with a frequency bandwidth of 4 GHz, such as from 77 GHz to 81 GHz, for example.

FIG. 2 is a function block diagram schematically illustrating an example configuration of the electronic device 1 according to an embodiment. The following describes an example of the configuration of the electronic device 1 according to an embodiment.

Frequency-modulated continuous-wave radar (hereinafter referred to as FMCW radar) is often used to measure distances and the like using millimeter-wave radar. In FMCW radar, a transmission signal is generated by sweeping the frequencies of the radio waves to be transmitted. Accordingly, in a millimeter-wave FMCW radar that uses radio waves in the 79 GHz frequency band, for example, the frequencies of the radio waves to be used have a frequency bandwidth of 4 GHz, such as from 77 GHz to 81 GHz, for example. Radar in the 79 GHz frequency band is characterized by having a wider usable frequency bandwidth than other millimeter/quasi-millimeter wave radars, such as radars in the 24 GHz, 60 GHz, and 76 GHz frequency bands, for example. The following describes such an embodiment as an example.

The radar scheme of the FMCW radar used in the present disclosure may include a fast-chirp modulation (FCM) scheme that transmits chirp signals on a shorter period than usual. The signal that the electronic device 1 generates is not limited to a signal of the FMCW scheme. The signal that the electronic device 1 generates may also be a signal of any of various schemes other than the FMCW scheme. A transmission signal sequence stored in any storage unit may be different depending on these various schemes. For example, in the case of a radar signal of the FMCW scheme described above, a signal of increasing frequency and a signal of decreasing frequency at each time sample may be used. Known technologies can be applied, as appropriate, for the various schemes described above, and thus a more detailed description is omitted.

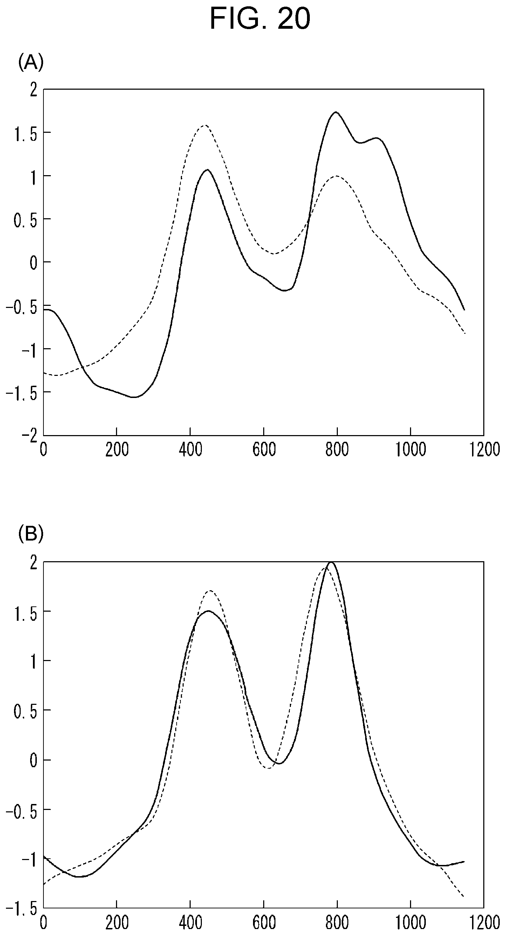

As illustrated in FIG. 2, in an embodiment, the electronic device 1 is provided with a signal processing unit 10. The signal processing unit 10 may be provided with a signal generation processing unit 11, a received signal processing unit 12, a heartbeat extraction unit 13, and a calculation unit 14. The heartbeat extraction unit 13 may execute processing to extract a micro-Doppler component, for example. The heartbeat extraction unit 13 may also execute processing to extract an envelope of the heart sound of the test subject 200. The calculation unit 14 may execute processing to calculate a heartbeat interval (RRI) of the test subject 200, for example. The calculation unit 14 may also execute processing to calculate the heartbeat of the test subject 200, for example. The calculation unit 14 may further execute processing to calculate a heart rate variability (HRV) of the test subject 200, for example. In this case, the calculation unit 14 may also execute processing to perform frequency analysis of time-series data on the extracted heartbeat interval of the test subject 200. The calculation unit 14 may also execute processing to calculate the heart rate variability of the test subject 200 on the basis of frequency analysis of time-series data on the heartbeat interval. The signal generation processing unit 11, received signal processing unit 12, heartbeat extraction unit 13, and calculation unit 14 will be further described later, as appropriate. In the present disclosure, the heart sound may refer to, for example, a chest vibration waveform (see FIG. 20 and the like) observed directly by radar, or to chest vibrations. The heartbeat refers to the pulsations themselves of the heart. A heartbeat interval, heart rate, or the like may be calculated from the movement of the heartbeat. The heartbeat interval may refer to the time interval between a pulsation of the heart and the next pulsation of the heart.

In an embodiment, the electronic device 1 is provided with a transmission DAC 21, a transmission circuit 22, a millimeter-wave transmission circuit 23, and the transmitting antenna 24 as the transmission unit. In an embodiment, the electronic device 1 is provided with the receiving antenna 31, a mixer 32, a reception circuit 33, and a reception ADC 34 as the reception unit. In an embodiment, the electronic device 1 need not include at least one of the functional units illustrated in FIG. 2, and may also include a functional unit other than the functional units illustrated in FIG. 2. The electronic device 1 illustrated in FIG. 2 may be formed using a circuit configured in basically the same and/or similar way as a common radar using electromagnetic waves in the millimeter-wave band or the like. On the other hand, in the electronic device 1 according to an embodiment, the signal processing by the signal processing unit 10 may include processing different from the processing performed by a common radar of the related art.

In an embodiment, the signal processing unit 10 provided in the electronic device 1 can control operations by the electronic device 1 as a whole, including control of each of the functional units that make up the electronic device 1. In particular, the signal processing unit 10 performs various processing with respect to signals handled by the electronic device 1. To provide control and processing power for executing various functions, the signal processing unit 10 may include at least one processor, such as a central processing unit (CPU) or a digital signal processor (DSP). The signal processing unit 10 may be realized entirely with a single processor, with several processors, or with respectively discrete processors. The processor may be realized as a single integrated circuit. An integrated circuit is also referred to as an IC. The processor may be realized as a plurality of communicatively connected integrated circuits and discrete circuits. The processor may be realized on the basis of any of various other known technologies. In an embodiment, the signal processing unit 10 may be configured as a CPU (hardware) and a program (software) executed by the CPU, for example. The signal processing unit 10 may include a storage unit (memory) required for operations by the signal processing unit 10.

The signal generation processing unit 11 of the signal processing unit 10 generates a signal to be transmitted from the electronic device 1. In the electronic device 1 according to an embodiment, the signal generation processing unit 11 may generate a transmission signal such as a chirp signal (transmission chirp signal). In particular, the signal generation processing unit 11 may generate signals (linear chirp signals) whose frequency varies periodically and linearly. For example, the signal generation processing unit 11 may generate chirp signals whose frequency increases periodically and linearly from 77 GHz to 81 GHz over time. As another example, the signal generation processing unit 11 may generate a signal whose frequency periodically and linearly increases (up-chirp) from 77 GHz to 81 GHz and then decreases (down-chirp) over time. The signal that the signal generation processing unit 11 generates may also be preset in the signal processing unit 10, for example. The signal that the signal generation processing unit 11 generates may also be stored in advance in any storage unit or the like in the signal processing unit 10, for example. Since chirp signals used in technical fields such as radar are already known, a more detailed description is simplified or omitted, as appropriate. The signal generated by the signal generation processing unit 11 is supplied to the transmission DAC 21. For this reason, the signal generation processing unit 11 may be connected to the transmission DAC 21.

The transmission DAC (digital-to-analog converter) 21 functions to convert a digital signal supplied from the signal generation processing unit 11 to an analog signal. The transmission DAC 21 may include a common digital-to-analog converter. The signal converted to analog by the transmission DAC 21 is supplied to the transmission circuit 22. For this reason, the transmission DAC 21 may be connected to the transmission circuit 22.

The transmission circuit 22 functions to convert the signal converted to analog by the transmission DAC 21 to an intermediate frequency (IF) band. The transmission circuit 22 may include a common IF-band transmission circuit. The signal processed by the transmission circuit 22 is supplied to the millimeter-wave transmission circuit 23. For this reason, the transmission circuit 22 may be connected to the millimeter-wave transmission circuit 23.

The millimeter-wave transmission circuit 23 functions to transmit the signal processed by the transmission circuit 22 as a millimeter wave (RF wave). The millimeter-wave transmission circuit 23 may include a common millimeter-wave transmission circuit. The signal processed by the millimeter-wave transmission circuit 23 is supplied to the transmitting antenna 24. For this reason, the millimeter-wave transmission circuit 23 may be connected to the transmitting antenna 24. The signal processed by the millimeter-wave transmission circuit 23 is also supplied to the mixer 32. For this reason, the millimeter-wave transmission circuit 23 may also be connected to the mixer 32.

The transmitting antenna 24 is a plurality of transmitting antennas arranged into an array. In FIG. 2, the configuration of the transmitting antenna 24 is illustrated in a simplified manner. The transmitting antenna 24 transmits a signal processed by the millimeter-wave transmission circuit 23 to the outside of the electronic device 1. The transmitting antenna 24 may include a transmitting antenna array used in a common millimeter-wave radar.

In this way, in an embodiment, the electronic device 1 is provided with a transmitting antenna (transmitting antenna 24) and can transmit a transmission signal (transmission chirp signal, for example) as a transmission wave from the transmitting antenna 24.

As an example, suppose the case in which an object such as the test subject 200 is present in the surroundings of the electronic device 1, as illustrated in FIG. 2. In this case, at least a portion of the transmission wave transmitted from the transmitting antenna 24 is reflected by an object such as the test subject 200. The at least a portion of the transmission wave transmitted from the transmitting antenna 24 that is reflected by an object such as the test subject 200 may be reflected toward the receiving antenna 31.

The receiving antenna 31 receives a reflected wave. The reflected wave may refer to at least a portion of a transmission wave transmitted from the transmitting antenna 24 that is reflected by an object such as the test subject 200.

The receiving antenna 31 is a plurality of receiving antennas arranged into an array. In FIG. 2, the configuration of the receiving antenna 31 is illustrated in a simplified manner. The receiving antenna 31 receives a reflected wave resulting from a transmission wave transmitted from the transmitting antenna 24 being reflected. The receiving antenna 31 may include a receiving antenna array used in a common millimeter-wave radar. The receiving antenna 31 supplies a received signal received as a reflected wave to the mixer 32. For this reason, the receiving antenna 31 may be connected to the mixer 32.

The mixer 32 converts a signal (transmission signal) processed by the millimeter-wave transmission circuit 23 and a received signal received by the receiving antenna 31 to an intermediate frequency (IF) band. The mixer 32 may include a mixer used in a common millimeter-wave radar. The mixer 32 supplies a signal generated as a synthesized result to the reception circuit 33. For this reason, the mixer 32 may be connected to the reception circuit 33.

The reception circuit 33 functions to perform analog processing on a signal converted to an IF band by the mixer 32. The reception circuit 33 may include a common reception circuit that performs conversion to an IF band. The signal processed by the reception circuit 33 is supplied to the reception ADC 34. For this reason, the reception circuit 33 may be connected to the reception ADC 34.

The reception ADC (analog-to-digital converter) 34 functions to convert an analog signal supplied from the reception circuit 33 to a digital signal. The reception ADC 34 may include a common analog-to-digital converter. The signal converted to digital by the reception ADC 34 is supplied to the received signal processing unit 12 of the signal processing unit 10. For this reason, the reception ADC 34 may be connected to the signal processing unit 10.

The received signal processing unit 12 of the signal processing unit 10 functions to perform various processing on a digital signal supplied from the reception DAC 34. For example, the received signal processing unit 12 calculates the distance from the electronic device 1 to an object such as the test subject 200 on the basis of a digital signal supplied from the reception DAC 34 (distance measurement). The received signal processing unit 12 also calculates the relative velocity of an object such as the test subject 200 relative to the electronic device 1 on the basis of a digital signal supplied from the reception DAC 34 (velocity measurement). The received signal processing unit 12 further calculates the bearing angle of an object such as the test subject 200 as seen from the electronic device 1 on the basis of a digital signal supplied from the reception DAC 34 (angle measurement). Specifically, I/Q converted data may be inputted into the received signal processing unit 12. By accepting the input of such data, the received signal processing unit 12 performs a fast Fourier transform (2D-FFT) in each of the distance (range) and velocity directions. The received signal processing unit 12 then performs false alarm suppression and fixed probability conversion by removing noise points through processing such as constant false alarm rate (CFAR). The received signal processing unit 12 performs angle-of-arrival estimation for points that satisfy the CFAR criterion to obtain the position of an object such as the test subject 200. The information generated as a result of the distance measurement, velocity measurement, and angle measurement by the received signal processing unit 12 may be supplied to the heartbeat extraction unit 13.

The heartbeat extraction unit 13 extracts information related to heartbeat from information generated by the received signal processing unit 12. The operations for extracting information related to heartbeat by the heartbeat extraction unit 13 will be further described later. The information related to heartbeat extracted by the heartbeat extraction unit 13 may be supplied to the calculation unit 14.

The calculation unit 14 performs various arithmetic processing, computational processing, and/or the like on information related to heartbeat supplied from the heartbeat extraction unit 13. The arithmetic processing, computational processing, and/or the like by the calculation unit 14 will be further described later. Various information resulting from the arithmetic processing, computational processing, and/or the like by the calculation unit 14 may be supplied to, for example, a communication interface 50. For this reason, the calculation unit 14 and/or signal processing unit 10 may be connected to the communication interface 50. Various information resulting from the arithmetic processing, computational processing, and/or the like by the calculation unit 14 may also be supplied to another functional unit other than the communication interface 50.

The communication interface 50 is configured to include an interface that outputs information supplied from the signal processing unit 10 to, for example, an external device 60. The communication interface 50 may output information on at least one of the position, velocity, and angle of an object such as the test subject 200 as a controller area network (CAN) or other type of signal, for example, to the external device 60 or the like. For example, information on at least one of the position, velocity, and angle of an object such as the test subject 200 may be supplied to the external device 60 or the like via the communication interface 50. For this reason, the communication interface 50 may be connected to the external device 60 or the like.

As illustrated in FIG. 2, in an embodiment, the electronic device 1 may be connected to the external device 60 in a wired or wireless manner via the communication interface 50. In an embodiment, the external device 60 may be configured to include a computer of any kind, a control device of any kind, and/or the like. In an embodiment, the electronic device 1 may be configured to include the external device 60. The external device 60 may be configured in a variety of ways according to the manner in which information on heartbeat and/or heart sound detected by the electronic device 1 is to be used. Accordingly, a more detailed description of the external device 60 is omitted.

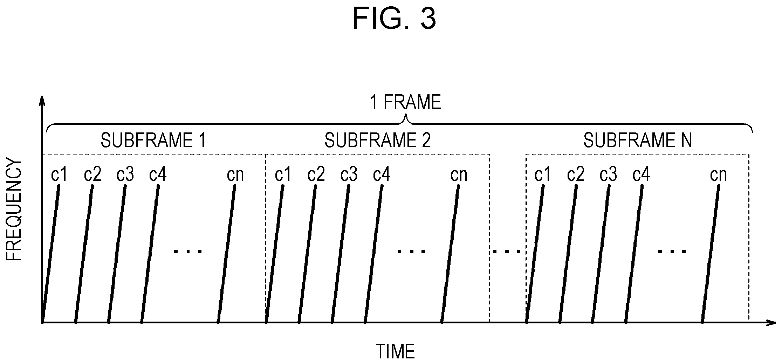

FIG. 3 is a diagram for describing an example of chirp signals generated by the signal generation processing unit 11 of the signal processing unit 10.

FIG. 3 illustrates the temporal structure of one frame in the case of using the fast-chirp modulation (FCM) scheme. FIG. 3 illustrates an example of a received signal of the FCM scheme. FCM is a scheme in which chirp signals illustrated as c1, c2, c3, c4, ..., cn in FIG. 3 are repeated at short intervals (for example, equal to or greater than the round-trip time of electromagnetic waves between the radar and the target as calculated from the maximum ranging distance). In FCM, the transmission and reception processing is often divided into subframe units as illustrated in FIG. 3 for convenient signal processing of a received signal.

In FIG. 3, the horizontal axis represents elapsed time, and the vertical axis represents frequency. In the example illustrated in FIG. 3, the signal generation processing unit 11 generates linear chirp signals whose frequency varies periodically and linearly. In FIG. 3, the chirp signals are indicated as c1, c2, c3, c4, ..., cn. As illustrated in FIG. 3, in each of the chirp signals, the frequency increases linearly over time.

In the example illustrated in FIG. 3, several chirp signals such as c1, c2, c3, c4, ..., cn are included as a single subframe. That is, subframe 1, subframe 2, and so on illustrated in FIG. 3 are each configured to include several chirp signals such as c1, c2, c3, c4, ..., cn. In the example illustrated in FIG. 3, several subframes such as subframe 1, subframe 2, ..., subframe N are included as a single frame (1 frame). That is, the 1 frame illustrated in FIG. 3 is configured to include N subframes. The 1 frame illustrated in FIG. 3 may be frame 1, followed by frame 2, frame 3, and so on. These frames are each configured to include N subframes, in the same and/or similar manner as frame 1. A frame interval of given length may also be included between frames. The single frame illustrated in FIG. 3 may be around 30-50 milliseconds long, for example.

In the electronic device 1 according to an embodiment, the signal generation processing unit 11 may generate a transmission signal as any number of frames. In FIG. 3, some of the chirp signals are omitted from illustration. The relationship between the time and frequency of a transmission signal generated by the signal generation processing unit 11 in this way may be stored in a storage unit or the like of the signal processing unit 10.

In this way, in an embodiment, the electronic device 1 may transmit a transmission signal formed from subframes including a plurality of chirp signals. In an embodiment, the electronic device 1 may transmit a transmission signal formed from frames including a given number of subframes.

The following describes the electronic device 1 as transmitting a transmission signal with a frame structure as illustrated in FIG. 3. However, the frame structure as illustrated in FIG. 3 is an example, and the chirp signals included in one subframes may be of any kind, for example. That is, in an embodiment, the signal generation processing unit 11 may generate subframes including any number of (for example, any plurality of) chirp signals. The subframe structure illustrated in FIG. 3 is also an example, and the subframes included in one frame may be of any kind, for example. That is, in an embodiment, the signal generation processing unit 11 may generate frames including any number of (for example, any plurality of) subframes. The signal generation processing unit 11 may generate signals of different frequency. The signal generation processing unit 11 may generate a plurality of discrete signals each having a different frequency f.

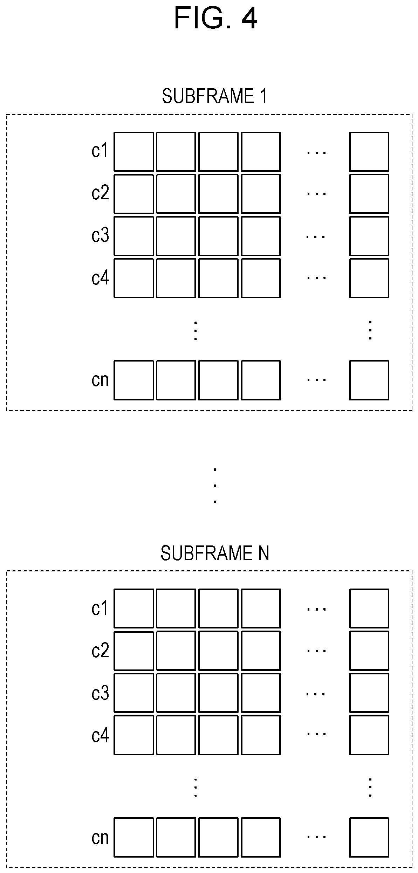

FIG. 4 illustrates another form of a portion of the subframes illustrated in FIG. 3. FIG. 4 is an illustration of samples of a received signal obtained by receiving the transmission signal illustrated in FIG. 3, as a result of performing processing, namely a two-dimensional fast Fourier transform (2D-FFT), in the received signal processing unit 12 (FIG. 2) of the signal processing unit 10.

As illustrated in FIG. 4, each of chirp signals c1, c2, c3, c4, ..., cn is stored in each of subframes such as subframe 1, ..., subframe N. In FIG. 4, each of the chirp signals c1, c2, c3, c4, ..., cn is formed from samples, which are indicated by the horizontally arrayed grid squares. The received signal illustrated in FIG. 4 is subjected to 2D-FFT, CFAR, integration signal processing on each subframe, and/or the like by the received signal processing unit 12 illustrated in FIG. 2.

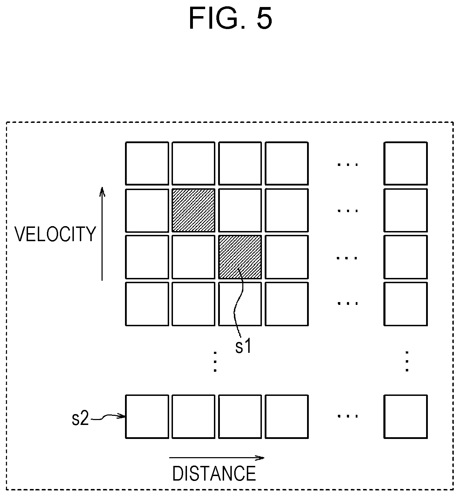

FIG. 5 illustrates an example of a point group in the range-Doppler (distance-velocity) plane calculated as a result of performing 2D-FFT, CFAR, and integration signal processing on each subframe in the received signal processing unit 12 illustrated in FIG. 2.

In FIG. 5, the horizontal direction represents range (distance), and the vertical direction represents velocity. The shaded grid square s1 illustrated in FIG. 5 illustrates a point group indicating a signal exceeding CFAR threshold processing. The non-shaded grid square s2 illustrated in FIG. 5 illustrates a bin (2D-FFT sample) with no point group, which did not exceed the CFAR threshold. Direction estimation is used to calculate the bearing, from the radar, of the calculated point group in the range-Doppler plane illustrated in FIG. 5, and the position and velocity in the two-dimensional plane are calculated as a point group indicating an object such as the test subject 200. Direction estimation may be calculated by a beamformer and/or a subspace method. Typical subspace method algorithms include MUltiple SIgnal Classification (MUSIC) and estimation of signal parameters via rotational invariant techniques (ESPRIT).

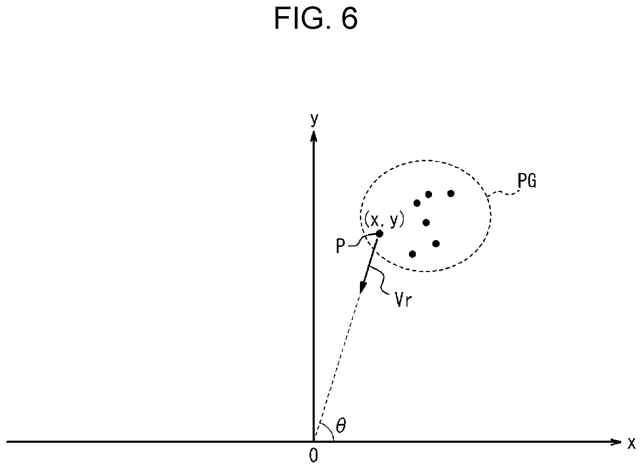

FIG. 6 illustrates an example of the result of the transformation of point group coordinates from the range-Doppler plane illustrated in FIG. 5 to the XY plane by the received signal processing unit 12 after performing direction estimation. As illustrated in FIG. 6, the received signal processing unit 12 can plot a point group PG in the XY plane. The point group PG contains points P. Each of the points P has an angle θ and a radial velocity Vr in polar coordinates.

The received signal processing unit 12 detects an object present in the range where a transmission wave T was transmitted, on the basis of at least one of the 2D-FFT or angle estimation results. The received signal processing unit 12 may perform object detection by performing, for example, clustering processing on the basis of respectively estimated information on distance, information on velocity, and angle information. Known algorithms used in clustering data include density-based spatial clustering of applications with noise (DBSCAN), for example. DBSCAN is an algorithm that performs density-based clustering. In the clustering processing, the average power of the points making up a detected object may be calculated, for example. The information on distance, information on velocity, angle information, and information on power pertaining to an object detected in the received signal processing unit 12 may be supplied to the external device 60 or the like via the communication interface 50, for example.

As above, the electronic device 1 may be provided with a transmitting antenna (transmitting antenna 24), a receiving antenna (receiving antenna 31), and the signal processing unit 10. The transmitting antenna 24 transmits a transmission wave T. The receiving antenna 31 receives a reflected wave R resulting from the transmission wave T being reflected. The signal processing unit 10 detects an object (an object such as the test subject 200, for example) that reflects the transmission wave T, on the basis of the transmission signal transmitted as the transmission wave T and the received signal received as the reflected wave R.

The following further describes direction estimation of an arriving wave by an antenna array of the electronic device 1 according to an embodiment.

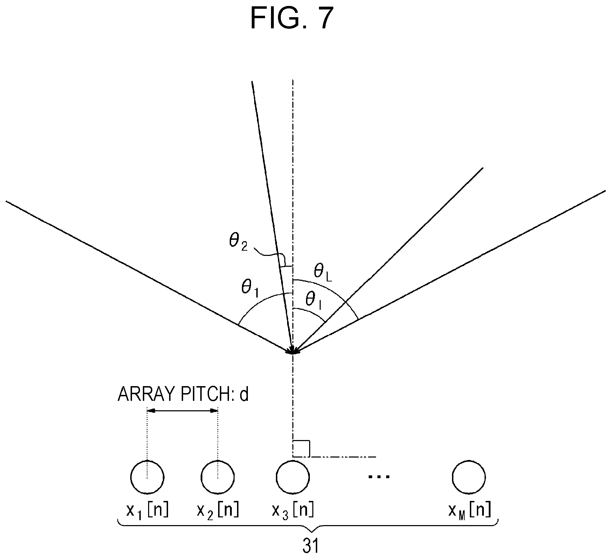

FIG. 7 is a diagram for describing the configuration of the receiving antenna 31 and the principle of direction estimation of an arriving wave by the receiving antenna 31 of the electronic device 1 according to an embodiment. FIG. 7 illustrates an example of the reception of radio waves by the receiving antenna 31.

As illustrated in FIG. 7, the receiving antenna 31 may be a linear arrangement of sensors such as receiving antennas. As illustrated in FIG. 7, in an embodiment, the receiving antenna 31 may be configured to include a plurality of receiving antennas in a linear array. In FIG. 7, the plurality of antennas x₁, x₂, x₃, ..., x_{M} that make up the receiving antenna 31 are illustrated by small circles. The receiving antenna 31 may include any plurality of antennas. As illustrated in FIG. 7, the plurality of antennas that make up the receiving antenna 31 are spaced apart from one another by an array pitch d. This type of sensor array, in which sensors (such as antennas, ultrasonic transducers, and microphones) corresponding to various physical waves are disposed in an array, is also referred to as a uniform linear array (ULA). As illustrated in FIG. 7, the physical waves (such as electromagnetic waves and sonic waves) arrive from various directions, such as θ₁ and θ₂, for example. Herein, θ₁ and θ₂ may refer to the angle of arrival described above. In this way, a sensor array like the receiving antenna 31 can estimate the direction of arrival (angle of arrival) by utilizing the phase difference that occurs in measurement values between sensors according to the direction of arrival of a physical wave. This type of technique for estimating the direction of arrival of a wave is also referred to as angle-of-arrival estimation or direction-of-arrival (DoA) estimation.

In the electronic device 1 according to an embodiment, at least one of the transmitting antenna 24 or the receiving antenna 31 may have a plurality of antennas in a linear arrangement. This allows for appropriate narrowing of directivity in the transmission and reception of radio waves in a millimeter-wave radar, for example. When transmitting a transmission wave, the direction of the transmission beam is often controlled by a beamformer. On the other hand, when receiving a reflected wave, the direction of arrival of the reflected wave is more often estimated by subspace methods (such as MUSIC and ESPRIT described above) than by a beamformer. With beamformers and subspace methods, for electromagnetic waves arriving from various directions in the ULA as illustrated in FIG. 7, a phase difference occurs in measurement values between sensors depending on the direction of arrival. Accordingly, this phase difference can be utilized to estimate the direction of arrival of a reflected wave.

The following further describes angle estimation in two directions of an arriving wave by an antenna array of the electronic device 1 according to an embodiment.

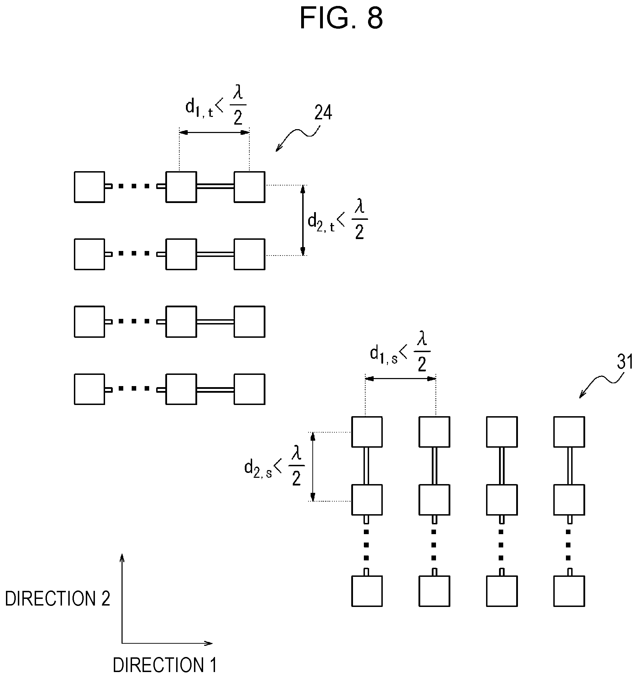

FIG. 8 illustrates an example arrangement of antennas for estimating the direction of arrival with respect to two orthogonal angles.

As illustrated in FIG. 8, in the electronic device 1 according to an embodiment, the transmitting antenna 24 and/or receiving antenna 31 may be configured to include an array of a plurality of patch antenna units.

In the transmitting antenna 24 illustrated in FIG. 8, one patch antenna unit may be configured to include a plurality of elements electrically connected in the direction of direction 1 illustrated in the diagram. In each of the patch antenna units, the plurality of elements may be electrically connected by wiring such as striplines on a board, for example. In each of the patch antenna units, the plurality of elements may be spaced apart from one another by a pitch d_{1,t} shorter than half the wavelength λ of the transmission wave. In FIG. 8, each of the patch antenna units may have any number of two or more elements electrically connected.

As illustrated in FIG. 8, the transmitting antenna 24 may include a plurality of patch antenna units arrayed in the direction of direction 2 illustrated in the diagram. The patch antenna units may be spaced apart from one another by a pitch d_{2,t} shorter than half the wavelength λ of the transmission wave. In an embodiment, the transmitting antenna 24 may include any number of two or more patch antenna units.

As illustrated in FIG. 8, in an embodiment, the receiving antenna 31 may be a variation of the arrangement of the plurality of elements in the transmitting antenna 24. That is, in the receiving antenna 31 illustrated in FIG. 8, one patch antenna unit may be configured to include a plurality of elements electrically connected in the direction of direction 2 illustrated in the diagram. In each of the patch antenna units, the plurality of elements may be electrically connected by wiring such as striplines on a board, for example. In each of the patch antenna units, the plurality of elements may be spaced apart from one another by a pitch d_{2,s} shorter than half the wavelength λ of the transmission wave. In FIG. 8, each of the patch antenna units may have any number of two or more elements electrically connected.

As illustrated in FIG. 8, the receiving antenna 31 may include a plurality of patch antenna units arrayed in the direction of direction 1 illustrated in the diagram. The patch antenna units may be spaced apart from one another by a pitch d_{1,s} shorter than half the wavelength λ of the transmission wave. In an embodiment, the receiving antenna 31 may include any number of two or more patch antenna units.

The elements included in the transmitting antenna 24 and the receiving antenna 31 may all be arranged in the same plane (on the surface layer of the same board, for example). The transmitting antenna 24 and the receiving antenna 31 may also be arranged in proximity to each other (monostatic). Direction 1 and direction 2 illustrated in FIG. 8 may be geometrically orthogonal.

With the transmitting antenna 24 and the receiving antenna 31 as illustrated in FIG. 8, the directivity of each of the transmitting antenna and the receiving antenna can be narrowed appropriately. By using the transmitting antenna 24 as illustrated in FIG. 8 to control the direction in which to transmit each transmission wave at each timing for transmitting a transmission wave (transmission signal), a beamformer can be realized for the direction of direction 2 illustrated in FIG. 8. By using the receiving antenna 31 as illustrated in FIG. 8, estimation of the direction of arrival of the reflected wave can be realized for the direction of direction 1 illustrated in FIG. 8. This enables estimation of the direction of arrival of a reflected wave with respect to two substantially orthogonal angles. Accordingly, a point group indicating an object such as the test subject 200 can be acquired three-dimensionally.

The following describes a technique for detecting the heartbeat of the test subject 200 by using the electronic device 1 according to an embodiment.

In an embodiment, the electronic device 1 measures (estimates) the heartbeat of the test subject 200 on the basis of the result of transmitting a millimeter-wave radar or other transmission wave toward the test subject 200 and receiving a reflected wave that was reflected in the chest area of the test subject 200 where the heart resides. As described above, the test subject 200 may be a human being, and may also be an animal. In this case, for example, the vibrations at the position where the test subject 200 is detected to be present by the radar can be filtered by frequency to extract components assumed to be the envelope curve of the heartbeat. Once the components assumed to be the envelope curve of the heartbeat are extracted, the peak-to-peak intervals of the envelope curve can be obtained as the heartbeat interval to roughly calculate the heartbeat interval. The approximation that peaks of the heartbeat envelope roughly correspond to R peaks in an electrocardiogram can be used. Therefore, the "heartbeat interval" may also be referred to as the RR interval (or RRI), like the terms used for electrocardiograms and the like.

The following is an examination of techniques for estimating the heartbeat interval of the test subject 200 from the result of performing the 2D-FFT, CFAR processing, and direction-of-arrival estimation described above and the like. The following describes the manner in which the heartbeat or body motion of a person is expressed as a result of the 2D-FFT performed in FIGs. 4 and 5.

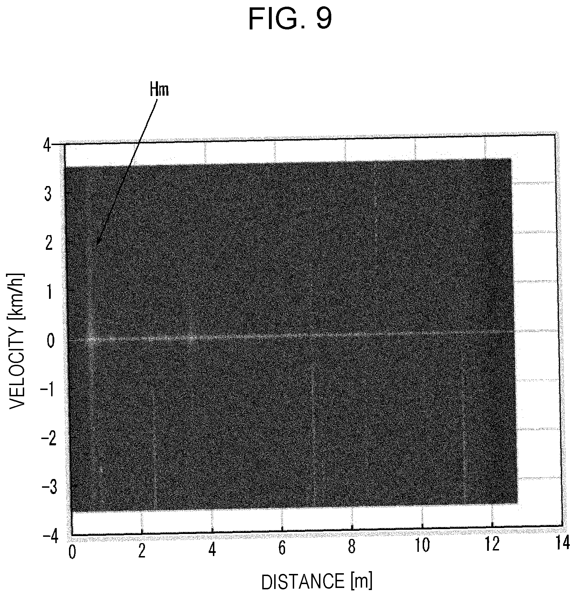

FIG. 9 illustrates an example of the result of receiving and performing 2D-FFT processing on reflected wave of a transmission wave transmitted toward the test subject 200. FIG. 9 illustrates a spectrum illustrating the heart sound and body motion of the test subject 200 as a result of the 2D-FFT. In FIG. 9, the horizontal axis represents distance (range), and the vertical axis represents velocity. In the electronic device 1 according to an embodiment, the signal processing unit 10 (for example, the heartbeat extraction unit 13) may extract a peak Hm as illustrated in FIG. 9, for example, as being body motion such as the heartbeat of the test subject 200. The spectral component indicated by the peak Hm in FIG. 9 includes not only the heart sound of the test subject 200 and the envelope curve of the heart sound, but also body motion. To extract the heartbeat interval, it may be necessary to extract body motion and the like, and thus frequency filtering is assumed to be performed, for example. The frequency filtering performed at this point is assumed to be, for example, a bandpass filter, high-pass filter, and/or low-pass filter targeting frequencies between 0.5 Hz and 10 Hz inclusive.

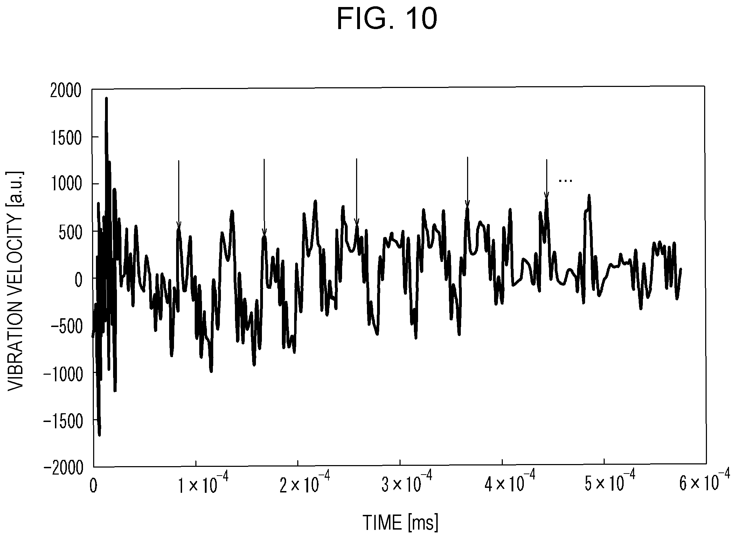

FIG. 10 is a diagram for describing a method of detecting peaks on the basis of the envelope waveform of the heart sound obtained by the frequency filtering described above. The graph illustrated in FIG. 10 illustrates an example of temporal changes in the envelope waveform of the heart sound extracted by the frequency filtering described above. The envelope waveform illustrated in FIG. 10 includes many peaks as illustrated in the diagram. On the other hand, the beating of the test subject 200 is known to fall within the range approximately from 50 to 130 beats per minute. Therefore, the rough heart sound interval of the test subject 200 can be calculated by selecting, from among the many peaks illustrated in FIG. 10, the peaks having a time interval from 0.4 seconds to 0.8 seconds, that is, the inverse of the number of beats per minute. For example, the peaks indicated by the downward-pointing arrows in FIG. 10 may be selected as the rough heart sound interval of the test subject 200.

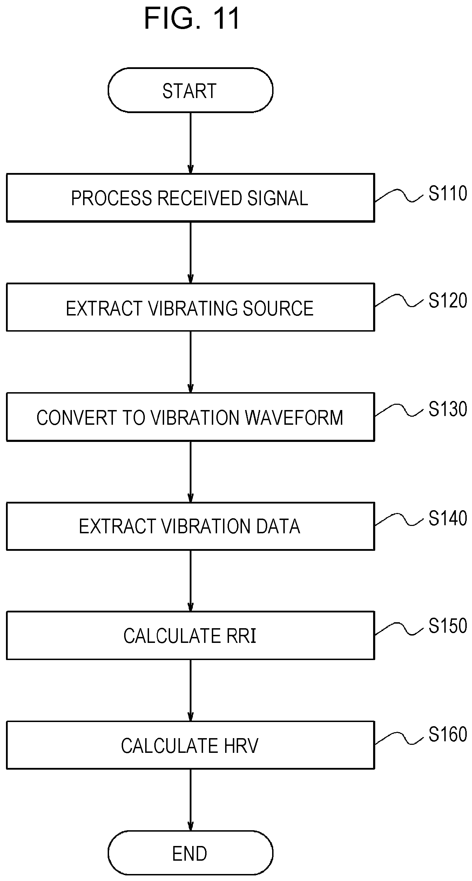

FIG. 11 is a flowchart illustrating an example of the heartbeat interval estimation operations described above. The following refers to FIG. 11 to summarize the heartbeat interval estimation operations described above.

FIG. 11 illustrates operations after the electronic device 1 according to an embodiment has received a reflected wave. That is, the operations illustrated in FIG. 11 presuppose that the electronic device 1 illustrated in FIG. 2 is transmitting a transmission wave (transmission signal) from the transmitting antenna 24. At least a portion of the transmission wave transmitted from the electronic device 1 is reflected by the test subject 200 (the chest portion thereof, for example) to become a reflected wave. The electronic device 1 illustrated in FIG. 2 is receiving such a reflected wave from the receiving antenna 31. From that point, the operations illustrated in FIG. 11 begin.

When the operations illustrated in FIG. 11 begin, in step S110, the signal processing unit 10 of the electronic device 1 processes the signal that is received (received signal). The signal processing performed in step S110 may include the 2D-FFT, CFAR processing, and/or direction-of-arrival estimation described above, for example. Such operations may be performed by the received signal processing unit 12 of the signal processing unit 10, for example.

In step S120, the signal processing unit 10 extracts a vibrating source from the information processed in step S110. The signal processing performed in step S120 may include processing for filtering the data that is the result of performing the 2D-FFT processing, for example. In step S120, the signal processing unit 10 may extract a spectral component at only the position where the test subject 200 is present. The position where the test subject 200 is present may be identified by any of various known techniques. Such operations may be performed by the heartbeat extraction unit 13 of the signal processing unit 10, for example.

In step S130, the signal processing unit 10 converts the processing result of the previous stage into a vibration waveform. The processing performed in step S130 may include processing to extract phase information from IQ data, for example. In step S130, the signal processing unit 10 may include processing to extract vibration data including heart sound from the spectral component of the 2D-FFT processing of the test subject 200 extracted in step S120. Such operations may be performed by the heartbeat extraction unit 13 of the signal processing unit 10, for example.

In step S140, the signal processing unit 10 extracts vibration data from the processing result of the previous stage. The processing performed in step S140 may include frequency filtering, for example. In step S140, the signal processing unit 10 may perform frequency filtering to extract a low-frequency signal including the envelope curve of the heart sound of the test subject 200. Such operations may be performed by the heartbeat extraction unit 13 of the signal processing unit 10, for example.

In step S150, the signal processing unit 10 calculates the RRI interval (RRI) of the test subject 200 by detecting peaks in the beating of the test subject 200 from the processing result of the previous stage and calculating the interval between the peaks. In step S150, the signal processing unit 10 may detect peaks in the low-frequency signal including the envelope curve of the heart sound of the test subject 200. In step S150, the signal processing unit 10 may also calculate and/or extract the interval between peaks. Such operations may be performed by the calculation unit 14 of the signal processing unit 10, for example. As above, in step S150, the signal processing unit 10 may extract the heart sound interval.

In step S160, the signal processing unit 10 may calculate the heart rate variability (HRV) of the test subject 200. In step S160, the signal processing unit 10 may calculate the HRV of the test subject 200 by calculating the spectral density of RRI time-series data. Calculating the power spectral density of RRI time-series data may involve using Welch's method or the like to perform frequency analysis on an RRI time-series waveform. Such operations may be performed by the calculation unit 14 of the signal processing unit 10, for example. In step S160, the signal processing unit 10 may also analyze the spectrum from the processing result of the previous stage.

As above, in an embodiment, the electronic device 1 performs frequency filtering on vibrations at the position where the test subject 200 is present to extract components thought to be the envelope curve of the heartbeat, and obtains the interval between peaks of the envelope curve as the heartbeat interval. In this way, in an embodiment, the electronic device 1 can calculate the rough heartbeat interval of the test subject 200.

In the calculation of the heartbeat interval as described above, several peaks may exist within the timespan of several tens of milliseconds, as illustrated in FIG. 10, for example. Consequently, selecting the peaks of the heartbeat involves some degree of uncertainty. For this reason, the precision of the calculated heartbeat interval will also have an error on the order of several tens of milliseconds. For example, in the calculation of the heartbeat interval described above, an error of around at least 20 ms occurs as compared to the instantaneous RRI acquired by an electrocardiograph. This precision makes it difficult to calculate the heart rate variability (HRV) and perform autonomic nervous system and/or sentiment analysis of a person. That is, in the calculation of the heartbeat interval described above, it is difficult to perform more sophisticated medical analysis by extracting the heart sound.

Accordingly, for example, a conceivable approach is to further cut the peaks on the high-frequency side by frequency filtering such that the time interval of the heartbeat is about 0.4 seconds to 0.8 seconds. However, since the precision of the information in the signal illustrated in FIG. 10 is not exceeded, reducing the error to around several tens of milliseconds is considered difficult.

In the calculation of the heartbeat interval described, the heart sound is not actually extracted. Consequently, with the technique described above, obtaining information that would contribute to medical examination (for example, auscultation for diagnosis and treatment) based on the acoustic properties of the heart sound itself is also considered difficult.

Accordingly, in an embodiment, the electronic device 1 further improves on the technique described above. In an embodiment, the electronic device 1 extracts the heart sound by radar using a high-frequency band at or above millimeter waves, for example, to thereby analyze the heart sound itself and extract an accurate heartbeat interval. The following describes such a technique.

In an embodiment, to extract the heart sound of the test subject 200 with high precision, the electronic device 1 performs appropriate signal processing on signals (chirp signals) received by the electronic device 1 in an appropriate sequence to thereby narrow down the subspace and subspace basis in which the signal components of the heart sound of the subject 200 exist. In an embodiment, the electronic device 1 may adopt different basis vectors in the linear space in which the signal of the heart sound of the test subject 200 exists. In this way, in an embodiment, the electronic device 1 may extract subspaces on the basis of coordinates for each while describing the space using an appropriate coordinate system. In an embodiment, the electronic device 1 can use such processing to search for subspaces in which the heart sound of the test subject 200 exists. In an embodiment, an appropriate coordinate system may be used to suit the purpose. For example, coordinate systems such as a coordinate system of the space in which the 2D-FFT processing is performed, a coordinate system of a time-series signal with temporal contraction of the chirp signals, a coordinate system based on a Fourier transform thereof, or a coordinate system based on continuous/discrete wavelets may be used.

In an embodiment, the electronic device 1 may execute characteristic processing like the following on a received signal according to a step-by-step procedure. The following summarizes characteristic processing by the electronic device 1 according to an embodiment. In an embodiment, the electronic device 1 executes three characteristic processes. Namely, in an embodiment, the electronic device 1 executes (1) Subspace extraction, (2) Envelope curve generation and best heart sound selection, and (3) Statistical signal processing for heartbeat interval calculation. The following describes each of these processes in greater detail.

### (1) Subspace extraction

In an embodiment, the electronic device 1 executes processing on a received signal to omit data considered unnecessary in a linear space. In an embodiment, the electronic device 1 may execute processing to omit unnecessary subspaces, thereby leaving the necessary subspaces. Specifically, processing like the following may be executed.

### (1-1) First stage: dimension reduction

The first stage involves applying an appropriate window function to the 2D-FFT processing of a chirp signal received by the electronic device 1, and using direction-of-arrival estimation to extract only a micro-Doppler component having a point group where the test subject 200 (a person or animal, for example) is present. Multiple window functions are generated around the rough position of the target and centered on a plurality of distance ranges, and these multiple window functions are applied. The processing to apply multiple window functions in this way is hereinafter referred to as "multi-window processing". One time-series signal of vibrations may be generated for one window function, that is, for one central distance range.

### (1-2) Second stage: subspace dimension reduction

The second stage involves executing, on a micro-Doppler signal, principal component analysis, singular value decomposition, and/or the like of a set of multiple time-series waveforms of vibrations extracted in the first-stage processing.

### (1-3) Third stage: subspace dimension reduction

The third stage involves executing frequency filtering by using at least one of the short-time Fourier transform, the continuous wavelet transform, or a bandpass filter.

### (1-4) Fourth stage: subspace dimension reduction

The fourth stage involves extracting the heart sound by performing multiresolution analysis via a discrete wavelet transform, using wavelet and scaling functions appropriate for heart sound.

### (2) Envelope curve generation and best heart sound selection

In an embodiment, to facilitate the processing of the extracted heart sound data, the electronic device 1 may execute processing to generate an envelope curve from the waveform of the extracted heart sound. In an embodiment, the electronic device 1 may further execute processing to select (extract) the best data from among the plurality of heart sound data. Specifically, processing like the following may be executed.

### (2-1) Envelope curve generation

An envelope curve is generated from the heart sound extracted in the above section (1) Subspace extraction. The envelope generation processing may use the processing of any of the continuous wavelet transform, a discrete wavelet transform, wavelet scattering coefficients, mel-frequency cepstral coefficients, moving variance, or the like.

### (2-2) Best heart sound selection

The best heart sound is selected from the generated envelope. The number of heart sounds generated by the above section (1) Subspace extraction is equal to the number of window functions. Consequently, the optimum heart sound is extracted (selected) from among the number of heart sounds equal to the number of window functions. A pre-trained learning classifier may be used to assess heart sound scores. In this case, the learning classifier may be, for example, an autoencoder, long short-term memory (LSTM), another type of neural network, a support vector machine, or a decision tree. The best heart sound is determined on the basis of the assessed scores. At the same time, accompanying calculation results may be acquired from the learning classifier. The learning classifier to be used may also be a linear support vector machine, simple perceptron, classifier using logistic regression, decision tree (classification tree), k-nearest neighbors algorithm, random forest, nonlinear support vector machine, neural network, or any combination of the above.

### (3) Statistical signal processing for heartbeat interval calculation

The heart sound interval is calculated by performing statistical processing on the generated best heart sound envelope. In most cases involving healthy subjects, the heart sound includes first heart sound (S1) and second heart sound (S2). The following two intervals exist when detecting the temporal interval between peaks of the heart sound within a certain time.
(i) First heart sound-Second heart sound interval (hereinafter also referred to as "S1-S2 interval").
(ii) Second heart sound-First heart sound interval (hereinafter also referred to as "S2-S1 interval").

A histogram or probability density distribution of these intervals will be a Gaussian mixture distribution or a simple Gaussian distribution. Consequently, an accurate heart sound interval can be assessed by using, for example, simple dynamic programming (DP) matching on the basis of information such as the above distribution.

According to an embodiment, the electronic device 1 can calculate the RR interval (RRI) with high precision by obtaining the envelope waveform of the heart sound obtained through the processing in each of the stages described above, detecting the positive and negative peaks therein, and appropriately calculating the interval between the peaks.

The following describes in greater detail operations by the electronic device 1 according to an embodiment.

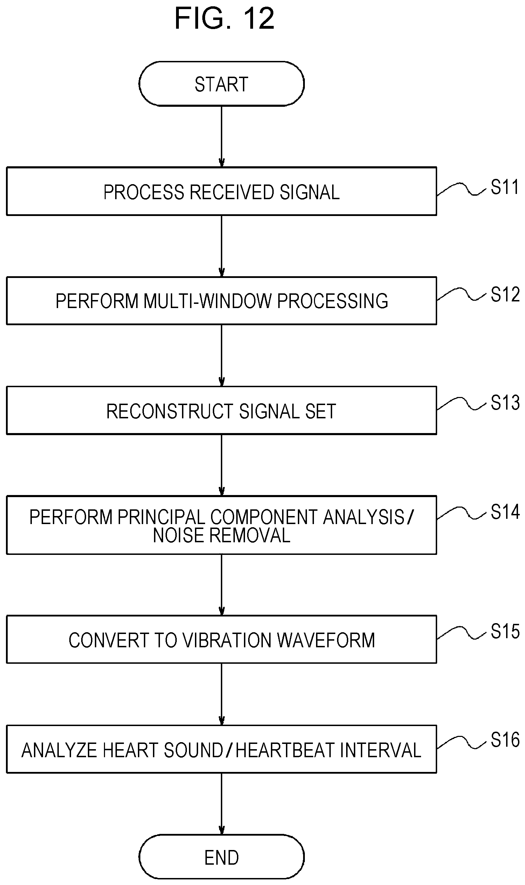

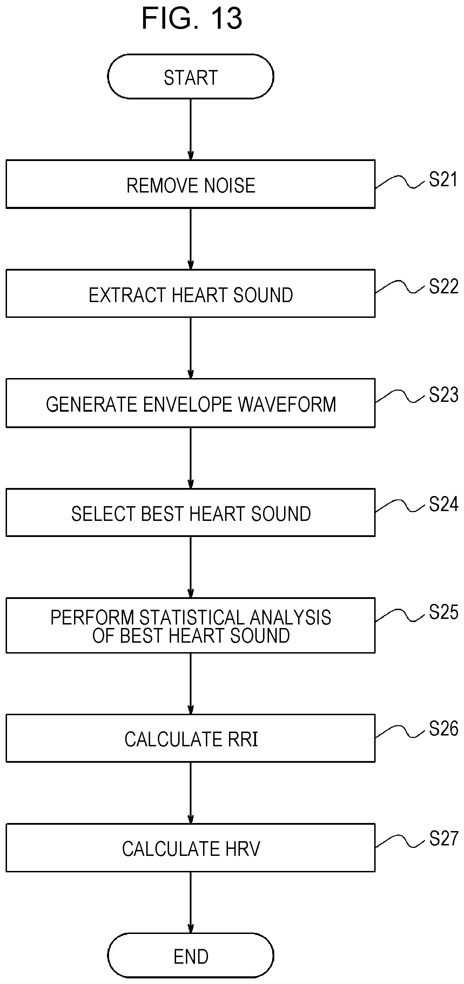

FIG. 12 is a flowchart illustrating an example of operations by the electronic device 1 according to an embodiment. FIG. 13 is a flowchart illustrating in greater detail an example of the operations in step S16 of FIG. 12. The following refers to FIGs. 12 and 13 to describe the flow of operations performed by the electronic device 1 according to an embodiment.

Step S11 illustrated in FIG. 12 can be performed in a manner similar to the operations in step S110 illustrated in FIG. 11. That is, when the operations illustrated in FIG. 12 begin, in step S11, the signal processing unit 10 of the electronic device 1 processes the signal that is received (received signal). The signal processing performed in step S11 may include the 2D-FFT, CFAR processing, and/or direction-of-arrival estimation described above, for example. Such operations may be performed by the received signal processing unit 12 of the signal processing unit 10, for example.

In step S12, the signal processing unit 10 performs multi-window processing. In step S12, the signal processing unit 10 may execute processing to determine candidate clusters of a person or animal and/or filtering processing (multi-window processing) on data processed using the 2D-FFT with multiple window functions applied.

In step S12, the signal processing unit 10 may perform multi-window processing for generating a plurality of heart sound candidates as a stage preceding the selection of the best heart sound candidate. The signal processing unit 10 may perform processing on the data processed using the 2D-FFT to gather a point group corresponding to a person or animal as a cluster. The signal processing unit 10 may perform multi-window processing by applying multiple window functions to a selected cluster. The multi-window processing performed at this point may include processing to generate data processed using the 2D-FFT that will serve as the basis of the plurality of heart sound candidates.

In step S12, the signal processing unit 10 may generate a plurality of heart sounds to serve as candidates by using an appropriate window function to extract only the region where the test subject 200 is present in the range-Doppler plane calculated by the 2D-FFT. In step S12, the signal processing unit 10 may provide a plurality of window functions as the appropriate window function. The signal processing unit 10 may use window functions such as the Hanning window, the Hamming window, and the Blackman-Harris window, for example.

In step S12, the signal processing unit 10 may execute detection of a vibrating source (target) that includes body motion, including the heart sound and/or respiration of the test subject 200, on the basis of the following first to third procedures, for example.

### (First procedure)

The signal processing unit 10 classifies point groups exceeding the CFAR threshold in the range-Doppler plane into predefined angular areas on the basis of the result of the direction-of-arrival estimation (see FIGs. 5 and 6). For example, provided that θ is the angle in the xy plane as illustrated in FIG. 6, angles may be classified into angular areas A to C as follows.
Area A: $- 10 deg < θ < 10 deg$
Area B: $- 20 deg < θ \leq 10 deg$
Area C: $10 deg \leq θ < 20 deg$

### (Second procedure)

The signal processing unit 10 applies clustering to point groups exceeding the CFAR threshold in the range-Doppler plane as indicated by S1 in FIG. 5, from among the groups in a certain one of the angular areas classified by the first procedure. A technique such as DBSCAN may be applied as the clustering, for example.

### (Third procedure)

Assuming that L clusters are processed by the second procedure, the signal processing unit 10 compares the deviation D_{dev}[l] in the number of bins in the Doppler direction for the l-th cluster with a certain threshold D_{dev,th}. As a result of the comparison, the signal processing unit 10 determines that only those with D_{dev}[l] ≥ D_{dev,th} are the test subject 200 and raises a flag HF[l]. That is, the signal processing unit 10 may perform processing as indicated by [Pseudocode 1] below, for example.

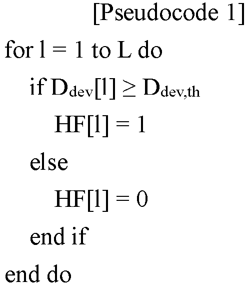

In step S13, the signal processing unit 10 reconstructs a set of chirp signals. In step S13, the signal processing unit 10 may reconstruct a set of chirp signals by performing an inverse fast Fourier transform (2D-IFFT) on the plurally generated 2D-FFT data.

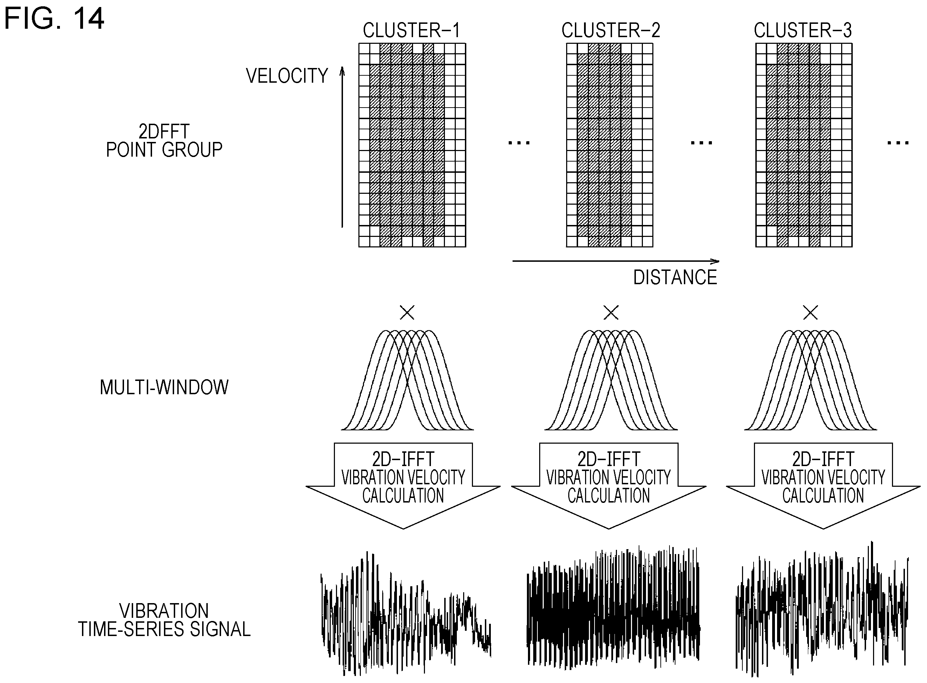

FIG. 14 is a diagram for describing multi-window processing by the electronic device 1 according to an embodiment. FIG. 14 is for describing multi-window processing in which a window function moved in a plurality of range directions is used to generate a plurality of time-series signals from a point group belonging to a cluster selected by the pseudocode 1 described above.

In the upper row of FIG. 14, the horizontal axis direction represents distance (range), and the vertical axis direction represents Doppler velocity. The upper row of FIG. 14 illustrates the results of clustering a point group of 2D-FFT data at a certain time. The upper row of FIG. 14 illustrates how the three clusters of cluster 1, cluster 2, and cluster 3 are generated as a result of clustering. The middle row of FIG. 14 conceptually illustrates how the signal processing unit 10 executes multi-window processing on each of cluster 1, cluster 2, and cluster 3. The middle row of FIG. 14 illustrates how the signal processing unit 10 performs multi-window processing by applying a window function to each of the clusters while shifting the window function a little at a time in the distance (range) direction. The lower row of FIG. 14 illustrates the results of the signal processing unit 10 calculating the vibration velocities by performing the 2D-IFFT on each of cluster 1, cluster 2, and cluster 3. The lower row of FIG. 14 illustrates how a vibration time-series signal is generated by performing the 2D-IFFT on each of the clusters. In actuality, a plurality of vibration time-series signals are obtained for each of the clusters illustrated in FIG. 14 as a result of applying the window function multiple times while shifting the window function a little at a time in the distance (range) direction).

In step S13 of FIG. 11, the signal processing unit 10 applies a plurality of time windows to a cluster formed from a point group selected as having the flag HF[l] = 1 by [Pseudocode 1] described above. This processing results in the generation of 2D-FFT data corresponding to each of the window functions. Let the 2D-FFT data generated by the l-th window function be expressed as in the following expression (1).
[Math. 1] ${\hat{S}}^{l}$

In this case, in step S13, the signal processing unit 10 can calculate a time-series signal waveform of the vibration velocity according to the following expression (2). The two-dimensional inverse fast Fourier transform (2D-IFFT) is used to reconstruct a set of chirp signals.
[Math. 2] ${S}^{l} = {F}^{- 1} \left({\hat{S}}^{l}\right) = \left[{s}_{1}^{l}, {s}_{2}^{l}, ⋯, {s}_{M}^{l}\right]$

Here, F⁻¹ represents the inverse fast Fourier transform, and M represents the number of chirps to be processed by the 2D-FFT.

The following describes an example with only one certain cluster, such as cluster 1 illustrated in FIG. 14.

In step S14, the signal processing unit 10 performs principal component analysis and/or noise removal with respect to the signal. Step S14 may involve singular value decomposition (SVD) as preprocessing to remove noise from the signal. This processing may be performed to remove noise that is low energy and/or low-level noise that is blended in due to the uncertainty of the Fourier transform.

In step S14, the signal processing unit 10 may perform singular value decomposition on the set S^{l} of received chirp signals extracted in the 2D-FFT plane. If U denotes a matrix of left singular vectors, Σ denotes a matrix of singular values arranged diagonally, and V denotes a matrix of right singular vectors, then the singular value decomposition of the set S^{l} of received chirp signals yields the following expression (3).
[Math. 3] ${S}^{l} = U Σ V * ∈ {ℂ}^{N \times M}$

In expression (3) above, the * appended to V represents the Hermitian transpose. N indicates the number of samples within a single chirp signal, and M indicates the number of chirp snapshots.

The signal processing unit 10 may limit (Uₑₓₜ) the number of row vectors in the rows of left singular vectors in expression (3) above. The signal processing unit 10 may limit (Σₑₓₜ) the number of diagonal elements in the diagonal matrix of singular values in expression (3) above. This removes unwanted noise signals and vibration components other than at the desired position. The resulting signal S^{l}ₑₓₜ projected into the subspace of the objective signal of S^{l} is indicated in the following expression (4).
[Math. 4] ${S}_{ext}^{l} = {U}_{ext} {Σ}_{ext} V * = \left[{s}_{ext , 1}^{l}, {s}_{ext , 2}^{l}, ⋯, {s}_{ext , M}^{l}\right] ∈ {ℂ}^{N \times M}$

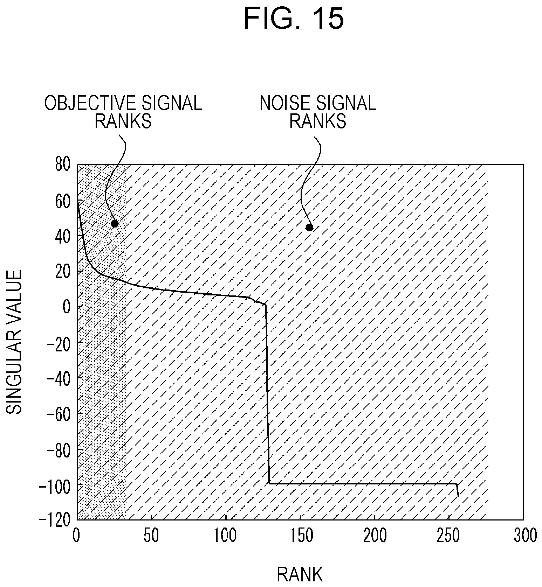

FIG. 15 illustrates the relationship between ranks representing an objective signal and a noise signal in singular value decomposition. FIG. 15 illustrates the SVD results arranged in descending order of singular value (which corresponds to energy), with rank on the horizontal axis and singular value on the vertical axis. The present disclosure adopts the idea that the objective signal is obtained when a signal at or above a certain singular value is extracted. In this case, the rank of the singular value that serves as the boundary between objective signal or not is the portion of the rank region at or below 30 indicated by the dark gray portion of the graph in FIG. 15. As illustrated in FIG. 15, the portion of the rank region at or below 30 is the region of the objective signal. In FIG. 15, the rank portion of the objective signal is between 1 and 30 inclusive. Accordingly, in the present disclosure, singular vectors corresponding to left and right singular values in the rank region between 1 and 30 inclusive may be extracted, for example.

In the example above, the rank portion of the objective signal is between 1 and 30 inclusive. The rank maximum value basically may be determined empirically. The rank maximum value (in the present disclosure, rank 30) may also be determined by a statistical technique. In the graph in FIG. 15, the singular values decrease suddenly at rank 130, and the signals of the ranks thereafter are basically noise. Accordingly, the subsequent rank signals may be unwanted.

In the graph in FIG. 15, the left and right singular vectors (vectors spanning the signal space) corresponding to the singular values of the area from rank 31 to rank 130 also have some objective signal components. These left and right singular vectors are assumed to be mainly due to unnecessary micro-vibrations and/or artificial noise (artifacts) generated by radar signal processing. Consequently, such elements may be cut off.

Once noise is removed by SVD in step S14, the signal processing unit 10 converts the result with the noise removed to a signal waveform (step S15). In step S15, the signal processing unit 10 may convert a beat signal (IQ data) to a vibration time-series signal.

In step S15, the signal processing unit 10 takes the sum in the column vector of each chirp signal on the basis of expression (4) above to obtain the vector indicated in the following expression (5).
[Math. 5] ${s}_{ext , sum}^{l} = \left[{\sum }_{m = 1}^{M} {s}_{ext , 1}^{l}, {\sum }_{m = 1}^{M} {s}_{ext , 2}^{l}, ⋯, {\sum }_{m = 1}^{M} {s}_{ext , N}^{l}\right]$

In step S15, the argument of s^{l}_{ext,sum} in the Gaussian plane and the derivative are taken to finally calculate a vector representing the vibration velocity, as indicated in the following expression (6).
[Math. 6] ${d}_{vib}^{l} = arg {s}_{ext , sum}^{l} , {v}_{vib}^{l} = \frac{d}{dt} {d}_{vib}^{l}$

In step S16, the signal processing unit 10 uses the obtained plurality of vibration waveforms to extract the heart sound and/or analyze the heartbeat interval. Specifically, in step S16, the signal processing unit 10 extracts the heart sound from the vibration waveforms indicated in expression (6) above. In step S16, the signal processing unit 10 may perform processing to generate a heart sound waveform, processing to calculate the heartbeat interval (RRI), and/or processing to calculate the heart rate variability (HRV). This allows the signal processing unit 10 to calculate the heartbeat interval. This processing may be executed on the plurality of vibration velocity waveforms that correspond to the window functions.

The processing in step S16 illustrated in FIG. 12 as described above may include more specifically at least a portion of the processing in each step from step S21 to step S27 illustrated in FIG. 13. The following describes in greater detail each step from step S21 to step S27 illustrated in FIG. 13.

In step S21 illustrated in FIG. 13, the signal processing unit 10 performs processing (denoising) to remove noise from a time-series waveform. The processing to remove noise may involve empirical Bayes methods, wavelet techniques such as the continuous wavelet transform (CWT), or the like.

More specifically, in step S21, the signal processing unit 10 performs preprocessing to further remove unwanted noise from the vibration velocity vector v^{l}_{vib} that corresponds to the l-th window function.

In step S21, the signal processing unit 10 may perform noise removal processing through band limiting using empirical Bayes methods and/or continuous wavelets. In step S21, the signal processing unit 10 may also perform noise removal processing through frequency subtraction using a noise profile for artifact noise and the like associated with the nonlinear processing from step S11 to step S14 illustrated in FIG. 12.

In step S22, the signal processing unit 10 extracts the waveform of the objective signal, that is, the waveform of the heart sound. In this case, for example, a technique involving discrete wavelets such as the maximal overlap discrete wavelet transform (MODWT) may be used.

More specifically, in step S22, the signal processing unit 10 may employ a wavelet waveform with a waveform similar to a heart sound waveform in the signal denoised as preprocessing, and execute multiresolution analysis using a discrete wavelet transform. In this way, in step S22, the signal processing unit 10 empirically extracts only the subspace at the level of multiresolution analysis where the heart sound of the test subject 200 is present. In this way, the signal processing unit 10 may extract the heart sound of the test subject 200 in step S22. Specifically, to improve the temporal resolution, the signal processing unit 10 may use maximal overlap multiresolution analysis (MODWT) or the like. Appropriate wavelet bases for heartbeat extraction may be symlet and Daubechies wavelets, for example. The order of the wavelets may be set case by case, as appropriate.

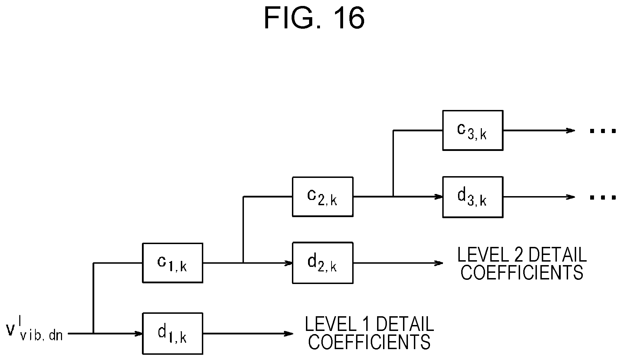

Provided that v^{l}_{vib,dn} is the signal resulting from performing the denoising process in step S21 on the signal velocity vector v^{l}_{vib}, the multiresolution analysis illustrated in FIG. 16 may be performed on the real part Re(v^{l}_{vib,dn}). FIG. 16 conceptually illustrates multiresolution analysis using a discrete wavelet transform. The signal processing unit 10 can obtain a heart sound waveform h by reconstruction with the empirically appropriate levels limited to j₀ ∈ N in the following expression (7).
[Math. 7] ${h}^{l} = \frac{1}{\sqrt{M}} \left[{\sum }_{k = 1}^{M - 1} {c}_{k} {ϕ}_{{j}_{0} , k}+{\sum }_{j = {j}_{1}}^{{j}_{0}} {\sum }_{k = 1}^{M - 1} {d}_{j , k} {ψ}_{j , k}\right] ∈ {ℝ}^{M}$

Here, Φ_{j0,k} and Ψ_{j,k} represent a scale function and a wavelet function, respectively, and cₖ and d_{j,k} represent a coefficient of each.

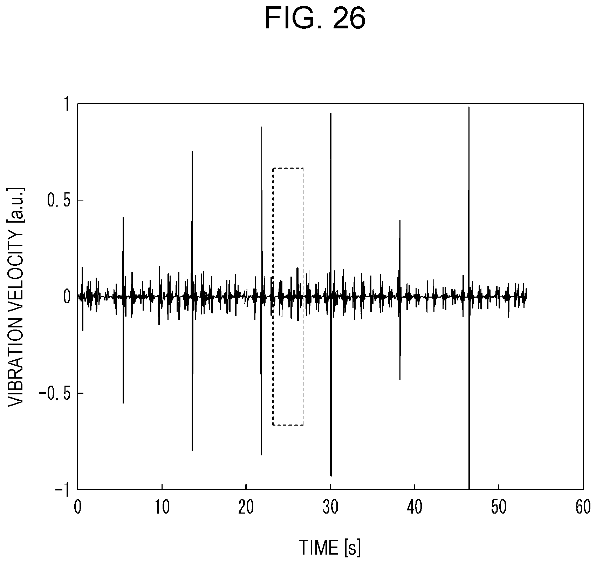

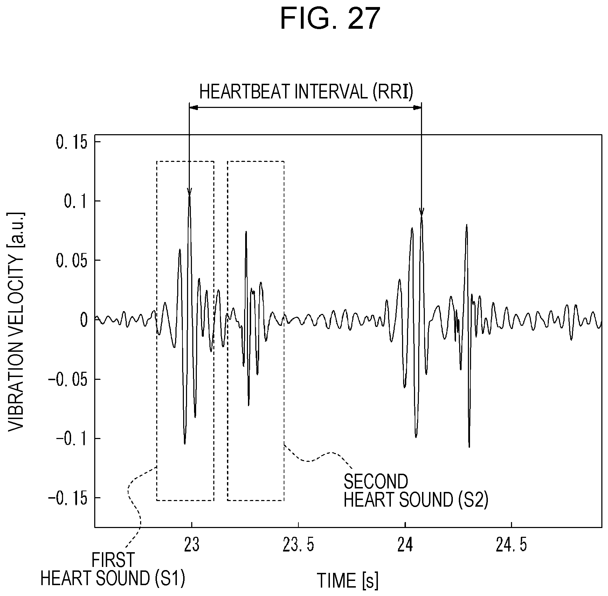

In this way, in an embodiment, the electronic device 1 can obtain a sound waveform h like the waveforms illustrated in FIGs. 26 and 27 described later, for example. The result can be utilized directly in heart sound diagnosis, treatment, and the like.

In step S23, the signal processing unit 10 generates an envelope waveform. In step S23, the signal processing unit 10 calculates a scalogram using the continuous wavelet transform and extracts an envelope waveform by one-dimensionalization thereof to detect (extract) energy peaks in the objective signal (heart sound waveform) extracted by step S22. At this point, the discrete/continuous wavelet transform, moving variance, Hilbert transform, and/or the like may be used.

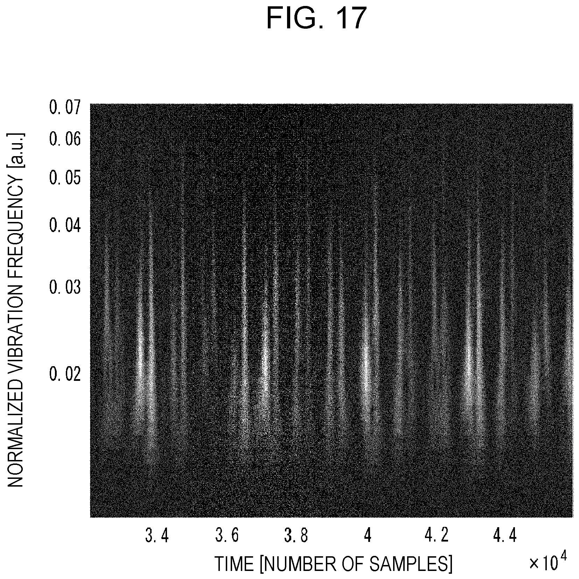

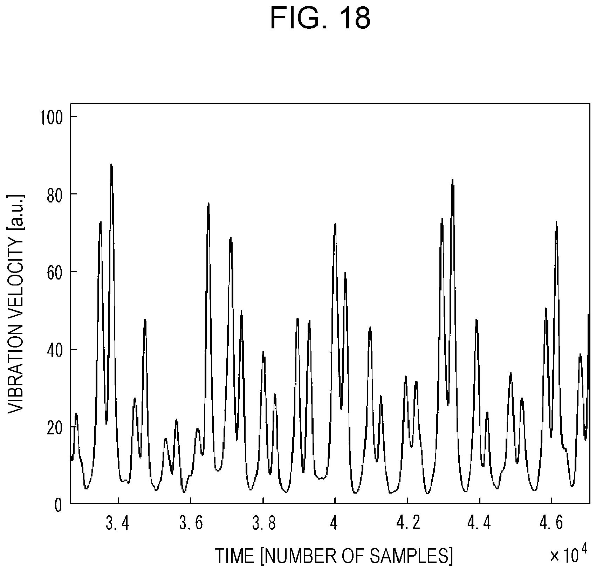

More specifically, in step S23, the signal processing unit 10 may obtain a scalogram using the continuous wavelet transform to obtain an envelope waveform of the heart sound waveform h¹ indicated in expression (7) above. The signal processing unit 10 takes the sum over a certain range from f₁ to f₂ on the frequency axis at each time in the scalogram. The signal processing unit 10 thus obtains a one-dimensional waveform s^{l}ₕ, as illustrated in FIGs. 17 and 18. FIGs. 17 and 18 are diagrams illustrating examples of a continuous wavelet transform analysis result. This analysis can be replaced by a discrete wavelet transform with due attention to resolution.

FIG. 17 illustrates a scalogram of the heart sound waveform h¹. FIG. 17 illustrates change over time in the frequency of normalized vibrations. The horizontal axis of FIG. 17 represents time in units of the number of samples, and the vertical axis of FIG. 17 represents the frequency of vibration normalized for each such unit of time.

FIG. 18 illustrates the result of performing one-dimensionalization processing using the continuous wavelet transform on the scalogram of the heart sound waveform h¹ illustrated in FIG. 17. FIG. 18 illustrates change over time in the vibration velocity. The horizontal axis of FIG. 18 represents time in units of the number of samples, and the vertical axis of FIG. 18 represents vibration velocity.

The matrix representing the absolute values of the scalogram is assumed to satisfy the following expression (8).
[Math. 8] ${S}_{h} ∈ {ℝ}^{M \times L}$

In this case, the one-dimensionalized envelope waveform e^{l}ₕ is represented as in the graph illustrated in FIG. 18. The signal processing unit 10 can calculate the envelope waveform e^{l}ₕ according to [Pseudocode 2] below.

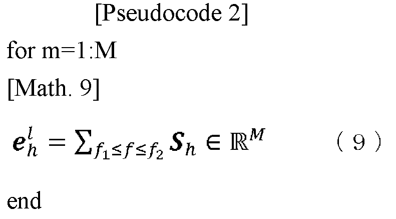

Here, e^{l}ₕ represents the vector of the one-dimensionalized envelope curve waveform, and f represents each frequency of the scalogram.

In step S24, the signal processing unit 10 selects the best heart sound. In step S24, the signal processing unit 10 may perform discrimination processing based on machine learning to extract the best heart sound envelope curve from a plurality of heart sound envelope waveforms extracted in step S23. Machine learning such as an autoencoder, long short-term memory (LSTM), another type of neural network, a support vector machine (SVM), or the like may be used.

More specifically, in step S24, the signal processing unit 10 selects the best from among the l vectors e^{l}ₕ obtained as heart sound envelope waveforms. To select the optimum e^{l}ₕ, the signal processing unit 10 may score a cost function of each to select the elh with the smallest cost function. The cost function is calculated using the error of reconstruction by an autoencoder.

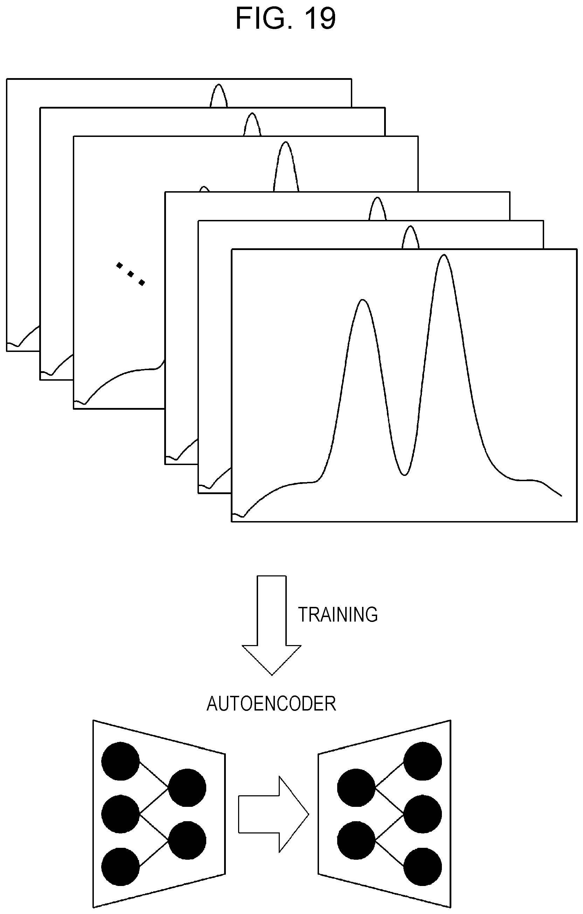

FIG. 19 and FIGs. 20(A) and 20(B) are diagrams for conceptually describing processing to classify and discriminate the best heart sound envelope curve. FIG. 19 conceptually illustrates how an autoencoder is trained on many pieces of training data. FIGs. 20(A) and 20(B) illustrate example of verifying the error in reconstructing data by an autoencoder. The vertical axis of FIGs. 20(A) and 20(B) represents arbitrary units determined by the digital scale of the signal processing.

In step S24, the signal processing unit 10 may perform heart sound envelope curve discrimination processing for selecting the best heart sound like in the following sub-steps S241-S243.

### <Sub-step S241>

In sub-step S241, the signal processing unit 10 may train the autoencoder by preparing many pieces of training data for datasets corresponding to first and second sound envelopes from among the heart sound envelopes illustrated in the upper part of FIG. 19. Let J denote the vector dimensionality of each piece of training data. The upper part of FIG. 19 illustrates the results of data augmentation and automatic extraction of individual proper heart sound envelope phonemes. By training on training data in this way, a trained autoencoder is generated, as illustrated in the lower part of FIG. 19.

### <Sub-step S242>

In sub-step S242, the signal processing unit 10 may execute reconstruction by the trained autoencoder, as illustrated in FIGs. 20(A) and 20(B). In this case, the signal processing unit 10 executes reconstruction by successively obtaining the envelope curve vectors e^{l}ₕ to be classified and discriminated as the i-th vector e^{l,i}ₕ trimmed to the length of J. The signal processing unit 10 calculates the mean absolute percentage error (MAPE) between the original envelope waveform vector e^{l,i}ₕ and the reconstructed envelope waveform vector indicated by the following expression (10).
[Math. 10] ${{e}_{h}^{l , ι}}^{˜}$

In this case, the signal processing unit 10 may successively calculate the MAPE while moving a window function for calculating the cost function, as indicated by [Pseudocode 3] below.

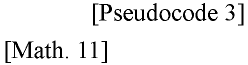

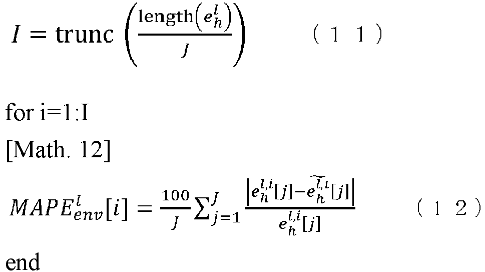

Here, length is a function representing the length of the signal vector, and trunc is a function that rounds off a number by removing digits to the right of the decimal point. In [Pseudocode 3] above, the processing is looped such that the last portion of e^{l}ₕ which falls short of the length J is discarded and not included in the calculation.

In FIGs. 20(A) and 20(B), one of either the solid-line graph or the dashed-line graph may illustrate a waveform based on detected data, while the other may illustrate a waveform based on reconstructed data. FIG. 20(A) illustrates a waveform that is not considered relatively good because the MAPE of the data exceeds a threshold, or in other words, the data cannot be predicted well (cannot be reconstructed properly). On the other hand, FIG. 20(B) illustrates a waveform that is considered relatively good because the MAPE of the data does not exceed a threshold, or in other words, the data can be predicted well (can be reconstructed properly).

### <Sub-step S243>

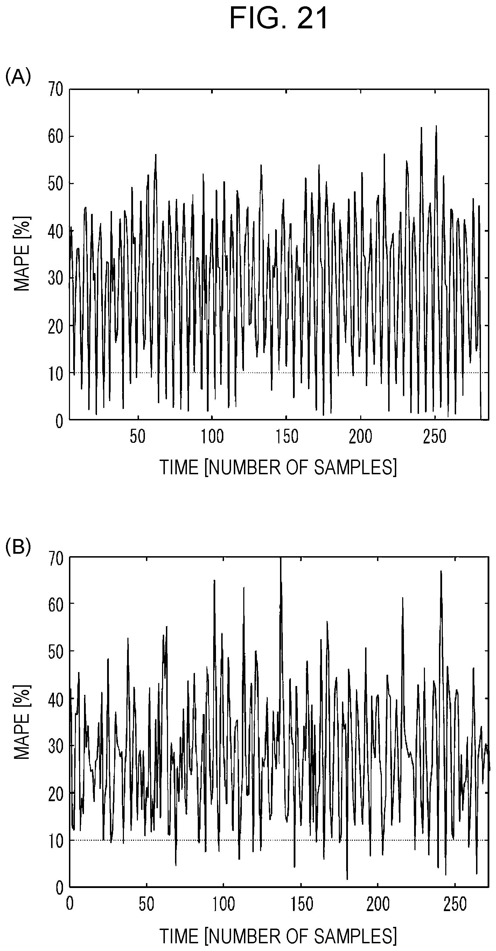

FIGs. 21(A) and 21(B) illustrate examples of the MAPE. FIGs. 21(A) and 21(B) are diagrams for explaining processing to extract the local minima of the MAPE in a result of reconstruction by an autoencoder to select the best heart sound. The horizontal axis of FIGs. 21(A) and 21(B) represents time in units of the number of samples, and the vertical axis of FIGs. 21(A) and 21(B) represents the MAPE (as a percentage). FIG. 21(A) illustrates an example of the MAPE^{l}ₑₙᵥ of the best heart sound envelope curve. FIG. 21(B) illustrates an example of the MAPE^{l}ₑₙᵥ of a heart sound envelope curve that is not the best.

In the electronic device 1 according to an embodiment, the signal processing unit 10 may set a certain threshold MAPEₜₕ on the MAPE as illustrated in FIG. 21(A) or 21(B). The signal processing unit 10 may calculate a cost function according to the local minima that go below the threshold MAPEₜₕ. As an example, the dotted line near the bottom of the graph in each of FIGs. 21(A) and 21(B) illustrates the line of the threshold when, for instance, MAPEₜₕ = 10. The cost function is assumed to be the number of local minima points at or below the threshold. When expressed mathematically, such a cost function becomes like the following expression (13).
[Math. 13] ${Cost}_{MAPE}^{l} = n \left(LocalMin\left({MAPE}_{env}^{l}\right)\leq {MAPE}_{th}\right)$

In expression (13) above, LocalMin is a function that lists local minima points. In the electronic device 1 according to an embodiment, the signal processing unit 10 may select the heart sound with the lowest cost function indicated in expression (13) above as the best heart sound. That is, the signal processing unit 10 may select the optimum heart sound number l_{best} as indicated in the following expression (14).
[Math. 14] ${e}_{h}^{{l}_{best}} = {argmin}_{l} {Cost}_{MAPE}^{l}$

MAPE^{l}ₑₙᵥ illustrated in FIG. 21(A) has relatively many local minima points that go below the threshold MAPEₜₕ (equal to 10%). Consequently, the signal processing unit 10 may deem the envelope curve illustrated in FIG. 21(A) to be an example of the best heart sound envelope curve. MAPE^{l}ₑₙᵥ illustrated in FIG. 21(B) has relatively few local minima points that go below the threshold MAPEₜₕ (equal to 10%). Consequently, the signal processing unit 10 may deem the envelope curve illustrated in FIG. 21(B) to be an example of a heart sound envelope curve that is not the best.

In step S25, the signal processing unit 10 statistically analyzes the best heart sound. In step S25, the signal processing unit 10 may extract positive and negative peaks of the best heart sound envelope curve selected in step S24 and perform statistical analysis of the peaks. This generates information for assessing the RRI. In step S25, the signal processing unit 10 may estimate the distribution of a Gaussian mixture model (GMM) or use an expectation-maximization algorithm (EM algorithm), variational Bayesian methods, or the like.

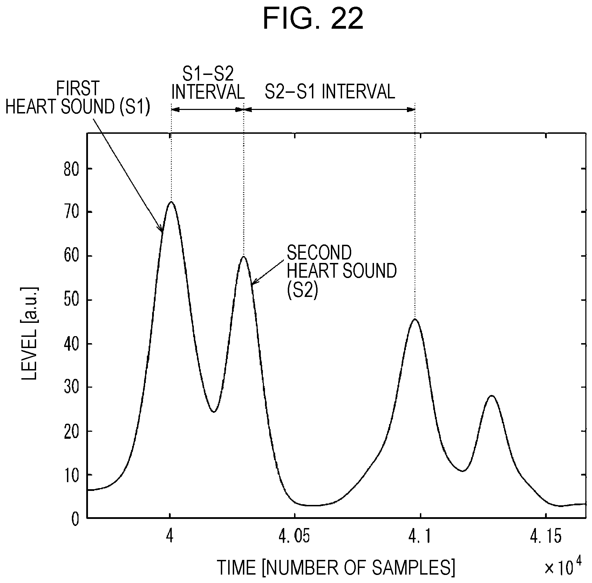

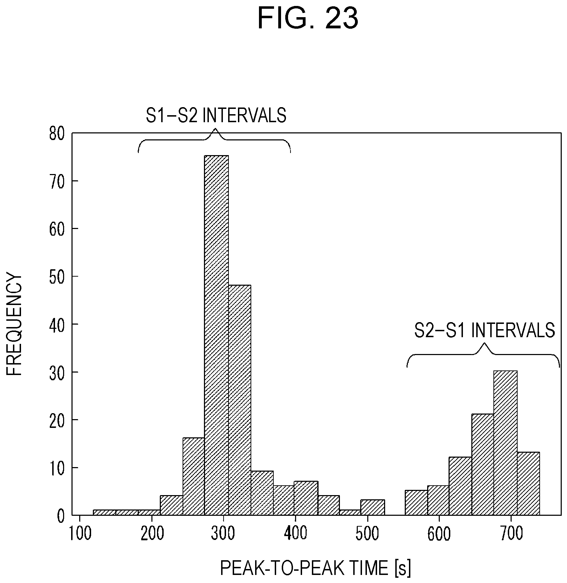

The following refers to FIGs. 22 and 23 to describe processing to generate a frequency distribution (histogram) of peak-to-peak intervals in a heart sound envelope curve. FIG. 22 illustrates an enlarged view of an example of two heart sound envelopes. The horizontal axis of FIG. 22 represents time in units of the number of samples, and the vertical axis of FIG. 22 represents signal level. FIG. 23 illustrates an example of a peak-to-peak interval histogram. The horizontal axis of FIG. 23 represents peak-to-peak time, and the vertical axis of FIG. 23 represents frequency.

FIG. 22 illustrates an example of the envelopes of first heart sound (S1) and second heart sound (S2). FIG. 22 illustrates the time interval between S1 and S2 (S1-S2 interval) and the time interval between S2 and S1 (S2-S1 interval). FIG. 23 illustrates a frequency distribution of S1-S2 intervals and S2-S1 intervals. FIG. 23 illustrates both histograms corresponding to each of the S1-S2 intervals and the S2-S1 intervals.

A probability density distribution normalized to this histogram is a Gaussian mixture distribution of two clusters. Consequently, to later estimate the RRI on the basis of this information, it may be necessary to estimate the Gaussian mixture distribution. A Gaussian mixture distribution is a distribution described by the linear sum of a plurality of Gaussian distributions, as indicated in the following expressions (15) and (16). As illustrated in the peak-to-peak interval histogram in FIG. 23, the peak-to-peak probability distribution of heart sound is a Gaussian mixture distribution.
[Math. 15] $p \left(x\right) = {\sum }_{k = 1}^{K} {π}_{k} N \left(\left.x\right|{μ}_{k},{Σ}_{k}\right) , 0 \leq {π}_{k} \leq 1 , {\sum }_{k = 1}^{K} {π}_{k} = 1$ [Math. 16] $N \left(\left.x\right|{μ}_{k},{Σ}_{k}\right) = \frac{1}{{\left(2π\right)}^{d / 2} {\left|{Σ}_{k}\right|}^{1 / 2}} exp \left(-\frac{1}{2}{\left(x-{μ}_{k}\right)}^{T}{\sum }_{k}^{- 1} \left(x-{μ}_{k}\right)\right)$

Here, x represents a d-dimensional data observation vector. Also, πₖ represents the mixture ratio of the Gaussian distributions. N represents a normal distribution. Also, µₖ represents the expected value of the k-th Gaussian distribution. Also, Σₖ represents the variance.

The following describes processing to estimate a Gaussian mixture distribution using variational Bayesian methods. This processing may include the following [First processing] and [Second processing]. The following describes [First processing] and [Second processing] in greater detail.

### [First processing]

An envelope curve of the heart sound (indicated by the following expression (17)) is extracted in individual time frames numbered k = 1, 2, ..., K at intervals of a certain length T. [Math. 17] ${e}_{h}^{{l}_{best}}$

The extraction of envelope curves of the heart sound indicated by expression (17) above may be performed while causing the envelope waveforms in the individual time frames to overlap one another.

In [First processing] above, the signal processing unit 10 divides the heart sound selected as the best heart sound (indicated by expression (17) above) into K time frames. That is, the signal processing unit 10 generates extracted envelope waveforms as indicated by the following expression (18).
[Math. 18] ${e}_{h}^{{l}_{best}} = \left[{e}_{h}^{{l}_{best} , 1}, {e}_{h}^{{l}_{best} , 2}, ⋯, {e}_{h}^{{l}_{best} , K}\right]$

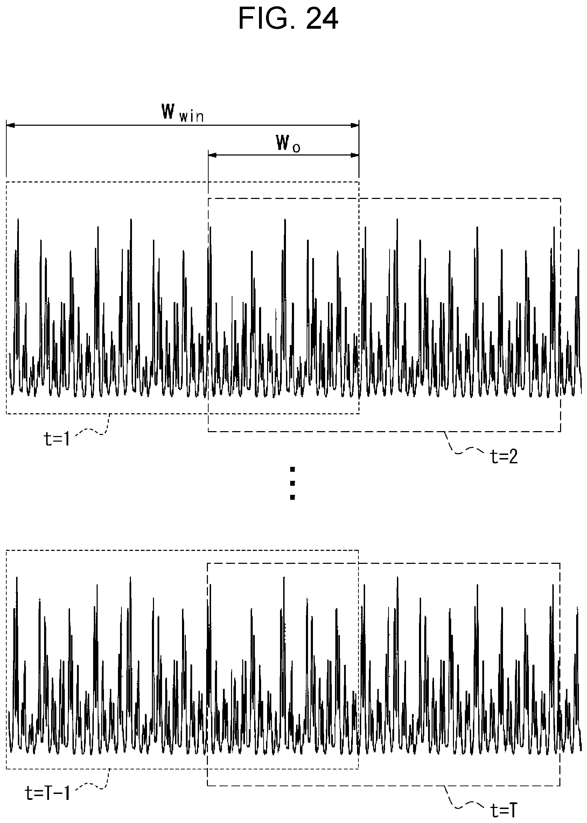

FIG. 24 is a diagram for describing processing to extract heart sound envelope curves. In the heart sound envelope curves illustrated in the upper part of FIG. 24, time windows for extracting the time frames t = 1 and t = 2 are illustrated. In the heart sound envelope curves illustrated in the lower part of FIG. 24, time windows for extracting the time frames t = T-1 and t = T are illustrated. Here, T and t are discrete times, each indicating a non-negative integer. In the heart sound envelope curves illustrated in the upper part of FIG. 24, the width w_{win} of the time window is illustrated along with the overlapping width wo. The moving step of the time window is wₛₜₑₚ = w_{win} - wₒ.

### [Second processing]

The Gaussian mixture distribution latent variables at time t = T is inputted as a prior distribution to update the latent variables at t = T+1 by variational Bayesian methods.

In [Second processing] above, the signal processing unit 10 uses the variational Bayesian method denoted below to perform Bayesian estimation, including latent parameters of the Gaussian mixture distribution, for the time frame t = k.

The following summarizes estimation by variational Bayesian methods. Variational Bayesian methods are techniques for estimating the posterior distribution of latent variables Z. Roughly speaking, variational Bayesian methods are techniques to approximate an actual posterior distribution p(Z|X) as a given approximate posterior distribution q(Z) by variational methods. In variational Bayesian methods, latent variables and variables are synonymous. In variational Bayesian methods, all variables are treated as latent variables whose true value cannot be calculated, or in other words, as random variables. The following summarizes variational Bayesian methods, with latent variables generalized.

In variational Bayesian methods, instead of minimizing the Kullback-Leibler divergence (KL divergence), which is described as in the following expression (19), an approximation is made by maximizing the following expression (20). Expression (20) is called the evidence lower bound (ELBO).
[Math. 19] ${D}_{KL} \left[q\left(Z\right)\left|p\left(Z\left|X\right.\right)\right.\right] = {\int }_{Z} q \left(Z\right) ln \frac{q \left(Z\right) p \left(X\right)}{p \left(X, Z\right)} d Z$ [Math. 20] $F \left[q\left(Z\right)\right] = {\int }_{Z} q \left(Z\right) ln \frac{p \left(X, Z\right)}{q \left(Z\right)} d Z$

Here, X indicates a matrix or vector representing a set of observation data. Z indicates a matrix or vector representing latent variables. Also, q indicates an approximate posterior probability distribution. Also, p(Z|X) and p(X, Z) indicate the true posterior distribution and joint distribution, respectively.

That is, the signal processing unit 10 can solve the following expression (21) to obtain the approximate posterior distribution q(Z) of the latent variables Z.
[Math. 21] $\hat{q} \left(Z\right) = {argmin}_{q \left(Z\right)} F \left[q\left(Z\right)\right]$

The mean field approximation indicated by the following expression (22) is ordinarily used to efficiently calculate expression (21) above.
[Math. 22] $q \left(Z\right) = {\prod }_{i = 1}^{I} {q}_{i} \left({Z}_{i}\right)$

The mean field approximation is an approximation based on the idea that the posterior probability distribution of latent variables collected into several groups is expressed by the product of each group, because the groups are all independent.

The signal processing unit 10 may also perform processing to solve the Euler-Lagrange equation indicated by the following expression (23) for expression (19) above, based on the condition in expression (22) above.
[Math. 23] $\frac{\partial f \left({q}_{i}\left({Z}_{i}\right),{Z}_{i}\right)}{\partial {q}_{i} \left({Z}_{i}\right)} = 0$

By solving the equation in expression (23) above, the signal processing unit 10 can obtain an approximate posterior distribution of the variables in the i-th group, as indicated by the following expression (24).
[Math. 24] ${q}_{i} \left({Z}_{i}\right) = A exp \left({\int }_{j \neq i} {q}_{j} \left({Z}_{j}\right) ln p \left(X, {Z}_{j}\right) d {Z}_{j}\right) = A exp \left({E}_{j \neq i}lnp\left(X, {Z}_{j}\right)\right)$

Here, f(qᵢ(Zᵢ), Zᵢ) is a function that satisfies the following expression (25).
[Math. 25] $F \left[q\left(Z\right)\right] = F \left[q, Z\right] = \int f \left({q}_{i}\left({Z}_{i}\right),{Z}_{i}\right) d Z$

Expression (24) means that the expected value of the logarithm of the joint probability of an observed value and a latent variable is taken by a latent variable other than itself.

The following describes a technique of utilizing the variational Bayesian method described thus far to estimate an actual Gaussian mixture distribution.

It may be necessary to group the latent variables of the approximate posterior distribution and perform the mean field approximation. To use expression (24) for updating the variational Bayes, Bayes' theorem is used to decompose the joint probability density distribution of the latent variables of the Gaussian mixture distribution into each probability distribution as indicated by the following expression (26), considering the dependencies of each of the latent variables.
[Math. 26] $p \left(X, z, π, μ, Σ\right) = p \left(X\left|Z\right.,μ,Σ\right) p \left(Z\left|π\right.\right) p \left(π\right) p \left(μ\left|Σ\right.\right) p \left(Σ\right)$

The latent variables of the Gaussian mixture distribution may be the Gaussian distribution mixture ratios π = [π₁, π₂, ..., π_{K}], the Gaussian distribution expected values µ = [µ₁, µ₂, ..., µ_{K}], the Gaussian distribution variances Σ = [Σ₁, Σ₂, ..., Σ_{K}], and a one-hot vector zₙ = [zₙ₁, zₙ₂, ..., z_{nK}] indicating to which Gaussian distribution n pieces of data belong, for the K Gaussian distributions. Bayes' theorem is used to decompose the joint probability density distribution of these latent variables of the Gaussian mixture distribution into each probability distribution as indicated by the following expression (26), with consideration for the dependencies of each of the latent variables. The decomposition indicated in expression (26) above may be performed after having used a graphical model to clarify the dependencies of the latent variables.

The conjugate prior distributions of the probability distributions serving as the factors in expression (26) above is set as in the following expressions (27) through (31). When the prior and posterior distributions for a likelihood function have the same and/or similar functional form, the prior and posterior distributions are referred to as the conjugate prior distributions for the likelihood function.
[Math. 27] $p \left(X\left|z, μ, Σ\right.\right) = {\prod }_{j = 1}^{J} {\prod }_{k = 1}^{K} N {\left({x}_{n}\left|{μ}_{k}, {Σ}_{k}^{- 1}\right.\right)}^{{z}_{nk}}$ [Math. 28] $p \left(z\left|π\right.\right) = {\prod }_{j = 1}^{J} {\prod }_{k = 1}^{K} {π}_{k}^{{z}_{nk}}$ [Math. 29] $p \left(π\right) = P \left(π\left|α\right.\right) = \frac{1}{B \left(α\right)} {\prod }_{k = 1}^{K} {x}_{k}^{{α}_{k} - 1}$ [Math. 30] $p \left(μ\left|Σ\right.\right) = {\prod }_{k = 1}^{K} N \left({μ}_{k}\left|m\right.,{\left(β{Σ}_{k}\right)}^{- 1}\right)$ [Math. 31] $p \left(Σ\right) = {\prod }_{k = 1}^{K} W \left({Σ}_{k}\left|W, ν\right.\right) = C \left(W, ν\right) {\left|Σ\right|}^{\frac{ν - D - 1}{2}} exp \left(-\frac{1}{2}tr\left({W}^{- 1}{Σ}_{k}\right)\right)$

Regarding expression (29), P(π|α) is the Dirichlet distribution with α = [α₁, α₂, ..., α_{K}] as parameters. Also, B(α) is the beta function extended to be multivariate. Regarding expression (31), W(Σₖ|W, v) is the Wishart distribution of Σₖ with v, D, and W as parameters. Also, v is the number of data vectors. D is the number of variable quantities. W is the covariance of Σₖ. C(W, v) is a constant calculated using the gamma function, as indicated by the following expression (32).
[Math. 32] $C \left(W, ν\right) = {\left|W\right|}^{- ν / 2} {\left[{2}^{\frac{νD}{2}}{π}^{\frac{D \left(D-1\right)}{4}}{\prod }_{d = 1}^{D} Γ \left(\frac{ν + 1 - d}{2}\right)\right]}^{- 1}$

The approximate posterior distribution is grouped by the one-hot vector z and all other variables, and expression (24) is defined specifically as in the following expression (33). [Math. 33] $q \left(z, π, μ, Σ\right) = q \left(z\right) q \left(π, μ, Σ\right)$

For expression (33) above, the variational Bayesian update formula in expression (24) above will be updated mutually for the two groups. The two groups may be as follows: Group 1: z; Group 2: π, µ, Σ.

Variational Bayesian methods, unlike EM algorithms and the like, do not intrinsically distinguish between latent variables and general variables. Consequently, in variational Bayesian methods, distinguishing between the expectation step (E-step) and the maximization step (M-step) has no particular significance. However, for convenience, the above two steps are defined as follows.
E-step: Take the expected values for Group 2 and update the approximate posterior distribution q(z) of z
M-step: Take the expected values for q(z) of Group 1 obtained in the E-step and update the approximate posterior distribution q(π, µ, Σ) of Group 2

The following describes the processing of a Gaussian mixture distribution from time t = T-1 to time t = T by variational Bayesian methods as signal processing performed the signal processing unit 10 in the electronic device 1 according to an embodiment. This processing may include the following five sub-steps S251-S255, with the E-step and M-step above inserted among these sub-steps.

### <Sub-step S251>

In sub-step S251, each latent variable is initialized (when T = 0), or the latent variables at t = T-1 are inherited as a prior distribution (when T ≥ 1). The latent variables of the posterior distribution calculated at time t = T are given as initial values at time t = T. The latent variables of the posterior distribution calculated at time t = T may be α|_{t=T}, β|_{t=T-1}, ν|_{t=T-1}, m|_{t=T-1}, and W|_{t=T-1}. The initial values at time t = T may be α₀|_{t=T}, β₀|_{t=T}, ν₀|_{t=T}, m₀|_{t=T}, and W₀|_{t=T}. The subscript 0 is added to indicate that the value is an initial value.

### <Sub-step S252>

In sub-step S251, the approximate posterior distribution q(z) of z is calculated by the E-step, and the result is used to calculate the burden ratio rₙₖ (posterior probability of the one-hot vector). The E-step may involve using expression (33) above to rewrite expression (24) above as in the following expression (34).
[Math. 34] $q \left(z\right) = A exp \left(E ln p\left(X,π,μ,Σ\right)\right)$

Expression (34) above is used to calculate the burden ratio rₙₖ (posterior probability of the one-hot vector) according to the following expressions (35) and (39).
[Math. 35] $ψ \left({α}_{k}\right) = \frac{d}{{dα}_{k}} ln Γ \left({α}_{k}\right)$ [Math. 36] $ln {{π}^{∨}}_{k} = ψ \left({α}_{k}\right) - ψ \left({\sum }_{k = 1}^{K} {α}_{k}\right)$ [Math. 37] $ln {{Σ}^{∨}}_{k} = {\sum }_{d = 1}^{D} ψ \left(\frac{{ν}_{k} + 1 - d}{2}\right) + ln \left({2}^{D}\left|{W}_{k}\right|\right)$ [Math. 38] ${ρ}_{nk} = {{π}^{∨}}_{k} {{Σ}^{∨}}_{k}^{1 / 2} exp \left[-\frac{D}{2 {β}_{k}}-\frac{{ν}_{k}}{2}{\left({x}_{n}-{m}_{k}\right)}^{T}{W}_{k}\left({x}_{n}-{m}_{k}\right)\right]$ [Math. 39] ${r}_{nk} = \frac{{ρ}_{nk}}{{\sum }_{i = 1}^{K} {ρ}_{nj}}$

### <Sub-step S253>

In sub-step S253, the M-step involves using the burden ratio rₙₖ to update the latent variables of the Gaussian mixture distribution (Group 2). Here, n is an index of the data. Also, k is an index of the Gaussian distributions. The M-step involves rewriting expression (24) above as in the following expression (40).
[Math. 40] $q \left(π, μ, Σ\right) = A exp \left(E ln p\left(X, z\right)\right)$

Expression (40) above and the following expressions (41) through (48) are used to update the parameters.
[Math. 41] ${N}_{k} = {\sum }_{n = 1}^{N} {r}_{nk}$ [Math. 42] ${\overline{x}}_{k} = \frac{1}{{N}_{k}} {\sum }_{n = 1}^{N} {r}_{nk} {x}_{n}$ [Math. 43] ${S}_{k} = \frac{1}{{N}_{k}} {\sum }_{n = 1}^{N} {r}_{nk} \left({x}_{n}-{\overline{x}}_{k}\right) {\left({x}_{n}-{\overline{x}}_{k}\right)}^{T}$ [Math. 44] ${\left.{α}_{k}={α}_{0}\right|}_{t = T + 1} + {N}_{k}$ [Math. 45] ${\left.{β}_{k}={β}_{0}\right|}_{t = T + 1} + {N}_{k}$ [Math. 46] ${\left.{ν}_{k}={ν}_{0}\right|}_{t = T + 1} + {N}_{k}$ [Math. 47] ${m}_{k} = \frac{1}{{β}_{k}} \left({\left.{\left.{β}_{0}\right|}_{t = T + 1}{m}_{0}\right|}_{t = T + 1}+{N}_{k}{\overline{x}}_{k}\right)$ [Math. 48] ${\left.{W}_{k}^{- 1}={W}_{0}^{- 1}\right|}_{t = T + 1} + {N}_{k} {S}_{k} + \frac{{\left.{β}_{0}\right|}_{t = T + 1} {N}_{k}}{{\left.{β}_{0}\right|}_{t = T + 1} + {N}_{k}} \left({\left.{\overline{x}}_{k}-{m}_{0}\right|}_{t = T + 1}\right) {\left({\left.{\overline{x}}_{k}-{m}_{0}\right|}_{t = T + 1}\right)}^{T}$

### <Sub-step S254>

In sub-step S254, a convergence determination is made by repeating sub-steps S252 and S253, and the processing in sub-steps S252 and S253 is ended. Specifically, convergence may be determined when the increment of the log-likelihood function or the increment of the ELBO between iteration steps falls to a set value or less, or when a maximum number of iterations is exceeded.

In sub-step S254, the ELBO indicated in expression (20) can be used as an indicator of the convergence determination. Rewriting expression (20) in terms of the estimation of the Gaussian mixture distribution yields the following expression (49).
[Math. 49] $F \left[q\left(z, π, μ, Σ\right)\right] = {\int }_{z} {\int }_{π} {\int }_{μ} {\int }_{Σ} q \left(z\right) q \left(π, μ, Σ\right) ln \frac{p \left(X, z, π, μ, Σ\right)}{q \left(z\right) q \left(π, μ, Σ\right)} d z dπ dμ d Σ$

For the increment between iterations of expression (49) above, a threshold ΔFₜₕ is set, the iteration index is taken to be l, and iteration of the variational Bayesian calculation is stopped when the following expression (50) is satisfied.
[Math. 50] ${F}^{l} \left[q\left(z, π, μ, Σ\right)\right] - {F}^{l - 1} \left[q\left(z, π, μ, Σ\right)\right] < {ΔF}_{th}$

The variational Bayesian calculation may also be stopped when the number of iterations exceeds a predetermined number.

### <Sub-step S255>

In sub-step S255, a Gaussian mixture model (GMM) is finalized for each latent variable by taking the expected value of each distribution as a representative value. Variational Bayesian methods calculate the posterior distribution of a Gaussian mixture distribution. Accordingly, it may be necessary to determine a representative value to ultimately determine the Gaussian mixture distribution. The Gaussian mixture distribution is determined by taking the expected values for π, µ, and Σ from among the latent variables. That is, the Gaussian mixture distribution is determined by taking the expected value of each of the above as representative values, as in the following expressions (51) through (53).
[Math. 51] $\overline{π} = \left[{\overline{π}}_{1}, {\overline{π}}_{2}, ⋯, {\overline{π}}_{K}\right] = E \left[π\right]$ [Math. 52] $\overline{μ} = \left[{\overline{μ}}_{1}, {\overline{μ}}_{2}, ⋯, {\overline{μ}}_{K}\right] = E \left[μ\right]$ [Math. 53] $\overline{Σ} = \left[{\overline{Σ}}_{1}, {\overline{Σ}}_{2}, ⋯, {Σ}_{K}\right] = E \left[Σ\right]$

From the above, the Gaussian mixture distribution at time t = T, as obtained from the representative values of the approximate posterior distribution, is like the following expressions (54) and (55). The Gaussian mixture distribution obtained in this way may be used in the next step S26.
[Math. 54] ${p}_{rep}^{T} \left(x\right) = {\sum }_{k = 1}^{K} {π}_{k} N \left(\left.x\right|{\overline{μ}}_{k},{\overline{Σ}}_{k}\right) , 0 \leq {π}_{k} \leq 1 , {\sum }_{k = 1}^{K} {π}_{k} = 1$ [Math. 55] $N \left(\left.x\right|{\overline{μ}}_{k},{\overline{Σ}}_{k}\right) , = \frac{1}{{\left(2π\right)}^{d / 2} {\left|{\overline{Σ}}_{k}\right|}^{1 / 2}} exp \left(-\frac{1}{2}{\left(x-{\overline{μ}}_{k}\right)}^{T}{\overline{Σ}}_{k}^{- 1}\left(x-{\overline{μ}}_{k}\right)\right)$

In actuality, in many cases involving healthy subjects, only the first sound (first heart sound (S1)) and the second sound (second heart sound (S2)) are prominent, as illustrated in FIGs. 22 and 23. In such cases, the expressions are simplified to K = 2. For test subjects such as those with abnormal heartbeats, a third sound and/or fourth sound may become prominent. In such cases, the expressions are evaluated for K = 3 or K = 4.

This completes [Second processing], and may also complete the processing in step S25.

In step S26, the signal processing unit 10 calculates the RR interval (RRI) on the basis of the statistical information about the heart sound interval generated in step S25.

More specifically, in step S26, the signal processing unit 10 may estimate the RRI by using the Gaussian mixture distribution (expressions (54) and (55) above) calculated in step S25. In this case, the signal processing unit 10 first estimates the distribution of the RRI by using expressions (54) and (55) above.

The following expression (56) holds for the reproductive property of a normal distribution, that is, for two random variables X and Y that follow a normal distribution.
[Math. 56] $X ∼ N \left({μ}_{X}, {Σ}_{X}\right) , Y ∼ N \left({μ}_{Y}, {Σ}_{Y}\right) \to X + Y ∼ N \left({μ}_{X}+{μ}_{Y},{Σ}_{X}+{Σ}_{Y}\right)$

Thus, by considering FIGs. 22 and 23 in regard to expressions (54) and (55) above, the distribution of the RRI is estimated as in the following expression (57). In expression (57), µ_{RRI} and Σ_{RRI} are as in the following expression (58).
[Math. 57] ${p}_{RRI}^{T} \left(x\right) = N \left(\left.x\right|{μ}_{RRI},{Σ}_{RRI}\right)$ [Math. 58] ${μ}_{RRI} = {\sum }_{k = 1}^{K} {u}_{k} , {Σ}_{RRI} = {\sum }_{k = 1}^{K} {Σ}_{k}$

If t^{peak} denotes a vectorization of the peak times of the heart sound envelope curve to be picked up in FIGs. 22 and 23, t^{peak} is indicated as in the following expression (59).
[Math. 59] ${t}^{peak} = \left[{t}_{1}^{peak}, {t}_{2}^{peak}, ⋯, {t}_{N}^{peak}\right]$

Provided that t^{targ} is a vector of non-negative integer multiples of the mean µ_{RRI} of the probability density distribution of the RRI defined in expressions (57) and (58) above, t^{targ} is indicated as in the following expression (60).
[Math. 60] ${t}^{targ} = \left[{μ}_{RRI},2{μ}_{RRI},⋯,L {μ}_{RRI}\right]$

Here, t^{targ} is an approximate target time to select a candidate corresponding to a point at which to take the RRI from t^{peak}. A technique similar to dynamic programming (DP) matching is performed between expressions (59) and (60) above to extract the peak points of the envelope curve of the heart sound considered to be the RRI.

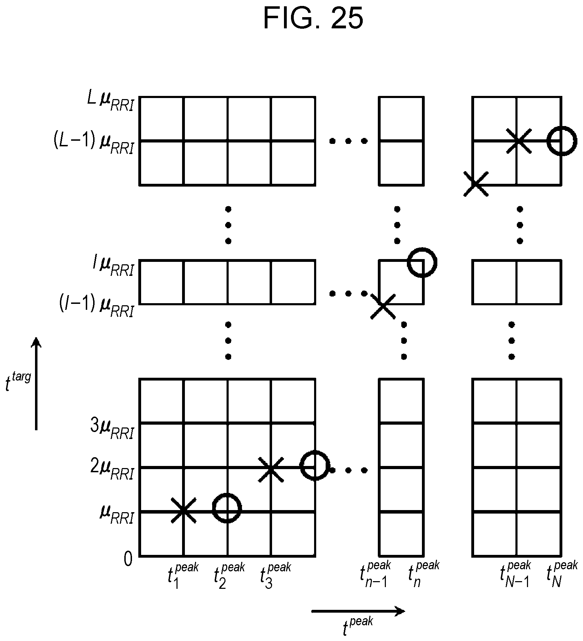

FIG. 25 is a diagram for describing DP matching for RRI estimation. In FIG. 25, the vectors t^{peak} are arranged horizontally and the vectors t^{targ} are arranged vertically. In FIG. 25, a circle indicates a match between t^{peak} and t^{targ}. In FIG. 25, an X indicates that matching was attempted according to [Pseudocode 4] below, but the matching was unsuccessful. Let m^{RRI} be a vector of flags indicating whether or not t^{peak} matched any element of t^{targ}. In this case, m^{RRI} can be expressed as in the following expression (61).
[Math. 61] ${m}^{RRI} = \left[{m}_{1}^{RRI}, {m}_{1}^{RRI}, ⋯, {m}_{N}^{RRI}\right] , {m}_{n}^{RRI} = \left(0, 1\right)$

Calculation processing for matching is performed according to [Pseudocode 4] below.

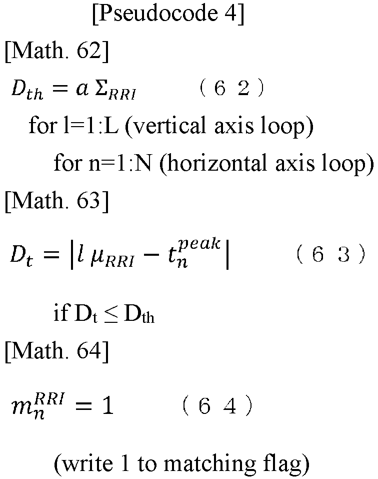

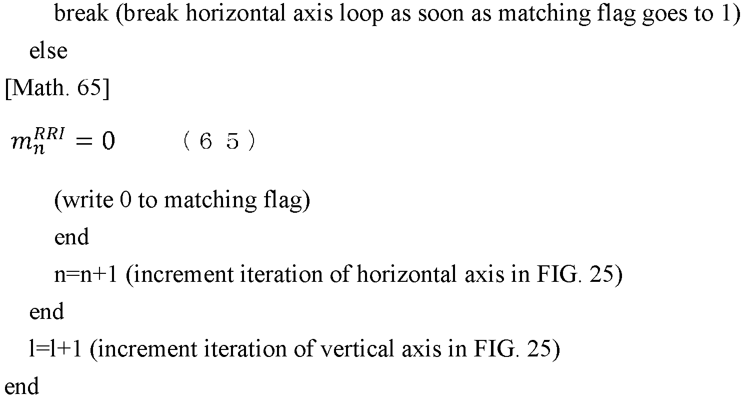

Expression (62) above means that the threshold Dₜₕ for the distance between t^{peak} and t^{targ} is determined by multiplying the variance Σ_{RRI} of the probability density distribution of the RRI indicated in the following expression (66) by a constant a.
[Math. 66] ${p}_{RRI}^{T} \left(x\right)$

Expression (63) above means processing to calculate the distance between the elements of t^{peak} and t^{targ} in a loop. That is, [Pseudocode 4] represents processing to extract as matching points the elements of t^{peak} that meet the condition indicated in the following expression (67).
[Math. 67] $l {μ}_{RRI} - {aΣ}_{RRI} \leq {t}_{n}^{RRI} \leq l {μ}_{RRI} + {aΣ}_{RRI}$

Let M be the number of nonzero elements of m^{RRI} as a calculation result of [Pseudocode 4], or in other words, the following expression (68) holds.
[Math. 68] ${\sum }_{n = 1}^{N} {m}_{n}^{RRI} = M$

By taking the Hadamard product (element product) of the matching flag m^{RRI} and t^{peak} and excluding zeros, a time sequence vector t^{RRI} of RRI points can be calculated. That is, the result of extracting only the points marked by a circle in the lattice illustrated in FIG. 25 is expressed as in the following expression (69).
[Math. 69] ${t}^{RRI} = RmZero \left({t}^{peak} °{m}^{RRI}\right)$

Here, RmZero is a function that removes zeros from the vector elements.

A vector Δt^{RRI} indicating RRI data is calculated by taking the difference between adjacent elements of t^{RRI}, as indicated in the following expression (70).
[Math. 70] $Δ {t}^{RRI} = \left[{Δt}_{1}^{RRI}, {Δt}_{2}^{RRI}, ⋯, {Δt}_{M - 1}^{RRI}\right] = \left[{t}_{2}^{RRI}-{t}_{1}^{RRI},{t}_{3}^{RRI}-{t}_{2}^{RRI},⋯,{t}_{M}^{RRI}-{t}_{M - 1}^{RRI}\right]$

The vector Δt^{RRI} indicating RRI data requires missing-value processing and smoothing processing. If the matching in the processing according to [Pseudocode 4] returns no point for which Dₜ ≤ Dₜₕ in the l-th iteration of the loops in FIG. 25, t^{RRI} will have missing values, which may result in outliers with extremely large values among the elements of Δt^{RRI}. Missing-value/outlier processing can be performed by, for example, allowing values to go up to the product of a certain multiplier b times the mean of Δt^{RRI} indicated in the following expression (71), and replacing a value with the mean if the value is larger than the product, as in Pseudocode 5 below.
[Math. 71] $Δ {\overline{t}}_{m}^{RRI}$

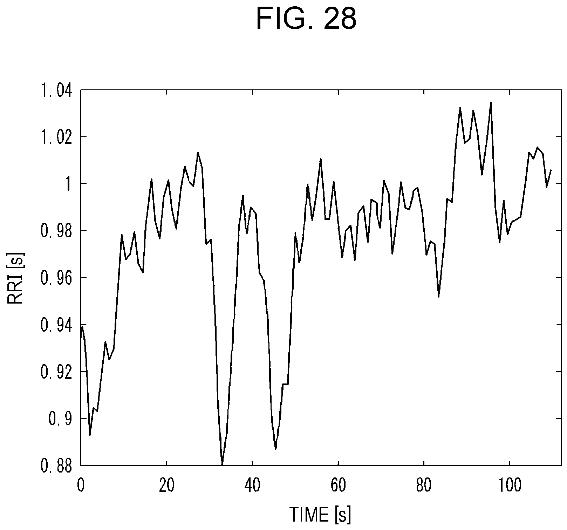

The RRI data subjected to missing-value/outlier processing in this way is given a dash and denoted as Δt^{'RRI}. This data is further acted upon by a moving average filter or the like, and the data Δt^{"RRI} subjected to smoothing as necessary can be used as the final RRI data. FIG. 28 illustrates an example of the Δt^{"RRI} data obtained in the above manner from FIGs. 26 and 27, which illustrate an example of a heart sound waveform. FIGs. 26, 27, and 28 illustrate examples of time-series waveforms of heart sound and an RRI obtained by the electronic device 1 to an embodiment. In FIGs. 26 and 27, the horizontal axis represents time, and the vertical axis represents vibration velocity. In FIG. 28, the horizontal axis represents time, and the vertical axis represents RRI. FIG. 26 illustrates a heart sound time-series waveform obtained by the electronic device 1 according to an embodiment. FIG. 27 illustrates an enlarged view of the timespan in the region enclosed by the dashed lines in FIG. 26.

As illustrated in FIG. 27, the first heart sound S1 and the second heart sound S2 can be identified clearly from the time-series waveform of the heart sound obtained by the electronic device 1 according to an embodiment. As illustrated in FIG. 27, the heartbeat interval RRI calculated from the interval between the first heart sound S1 and the second heart sound S2 can also be identified clearly from the time-series waveform of the heart sound obtained by the electronic device 1 according to an embodiment. Strictly speaking, the RRI and the heartbeat interval are not the same, but are considered to approximately match. Accordingly, in the present disclosure, the RRI and the heartbeat interval are described as indicating the same thing.

The result illustrated in FIG. 28 is calculated on the basis of the accurately extracted heart sound in FIGs. 26 and 27. Accordingly, the result illustrated in FIG. 28 is precise to within a few milliseconds.

In step S27, the signal processing unit 10 calculates the heart rate variability (HRV). In step S27, the signal processing unit 10 may calculate the power spectral density (PSD) by performing frequency analysis on the RRI time-series waveform. The frequency analysis of the RRI time-series waveform may involve using Welch's method, for example.

More specifically, in step S27, the signal processing unit 10 may calculate the FFT and power spectrum of Δt^{"RRI} estimated by step S26 according to Welch's method or the like, that is, by temporally overlapping window functions. The signal processing unit 10 can thus obtain the power spectral density (PSD) of the RRI.

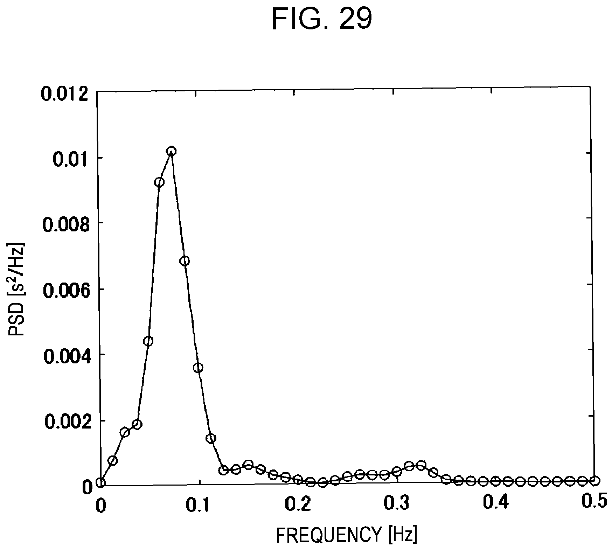

FIG. 29 illustrates an example of the power spectral density (PSD) of the RRI obtained by the signal processing unit 10. In FIG. 29, the horizontal axis represents frequency, and the vertical axis represents power spectral density (PSD). The method of calculating the power spectrum, such as Welch's method, may employ any known general method. Accordingly, a more detailed description of the method of calculating the power spectrum, such as Welch's method, is omitted.

FIG. 29 illustrates an example of the power spectral density calculated using Welch's method from the RRI time-series waveform illustrated in FIG. 28. According to an embodiment, the electronic device 1 can calculate the power spectral density of the RRI on the basis of the RRI that is precise to within a few milliseconds. Thus, according to an embodiment, the electronic device 1 can also accurately calculate the component of the heartbeat PSD from 0.15 Hz to 0.4 Hz, known as the HF component.

As described above, according to an embodiment, the electronic device 1 can obtain a detailed heart sound waveform as illustrated in FIGs. 26 and 27, accurate RRI time-series data as illustrated in FIG. 28, and the power spectral density (PSD) of the heartbeat interval as illustrated in FIG. 29, for example. According to an embodiment, the electronic device 1 may detect the heartbeat of a human body or the like with good precision by transmitting and receiving radio waves. Consequently, according to an embodiment, the electronic device 1 can detect weak vibrations, such as the heartbeat of a human body, with good precision by transmitting and receiving radio waves, such as millimeter waves for example, and is anticipated to be useful in a wide variety of fields.

In the examples in FIGs. 26 through 29, only the first heart sound S1 and second heart sound S2 prominent in healthy subjects are described. However, according to an embodiment, the electronic device 1 can also perform processing in a similar manner in the case of a third heart sound and/or fourth heart sound occurring as a result of an abnormal heartbeat.

### (Other embodiments)

The following describes other embodiments.

In another embodiment, the electronic device 1 according to an embodiment may calculate the heart rate from the RRI data rather than obtaining the PSD from the RRI data. In this case, the signal processing unit 10 of the electronic device 1 may execute processing to calculate the heart rate from the RRI data in step S27 illustrated in FIG. 13, for example. In this case, the signal processing unit 10 may perform, for example, the following processing 1 through 3 in step S27.
1. Low-pass filter (passing frequencies of around 1 Hz or lower) processing
2. Processing to take the reciprocal of the RRI
3. Processing to round off the reciprocal taken in processing 2 above to a natural number

The above processing allows the heart rate to be calculated from the RRI. In an embodiment, the electronic device 1 according to an embodiment may perform, for example, LPF processing, reciprocal calculation, natural number rounding processing, and/or the like as the processing in step S27 illustrated in FIG. 13.

In another embodiment, the electronic device 1 according to an embodiment may modify the processing indicated in step S24 illustrated in FIG. 13 when performing the processing illustrated in FIG. 13 and the processing 1 through 3 described above. For example, in an embodiment, the electronic device 1 may execute an autoencoder, template matching based on a cross-correlation function, classification and discrimination by LSTM, and/or classification and discrimination of other-dimensional vectors by an SVM as step S24 illustrated in FIG. 13.

In another embodiment, the electronic device 1 according to an embodiment may perform the processing in step S25 by an EM algorithm in processing like in FIG. 13. However, EM algorithms are not Bayesian estimation methods. Thus, EM algorithms do not have a mechanism for calculating a posterior distribution from a prior distribution, and when used to estimate a Gaussian mixture distribution, the variables π, µ, and Σ that describe the Gaussian mixture part are point estimates.
Consequently, when executing step S25 illustrated in FIG. 13 by an EM algorithm, one of the following approaches is taken.
- Perform Gaussian mixture distribution estimation by the EM algorithm for each time frame individually, and do not consider relationships between time frames.
- After having performed Gaussian mixture distribution estimation by the EM algorithm for each time frame individually, a moving average before and after may be taken or time-series filtering with a Kalman filter may be performed for each of the Gaussian mixture distribution variables π, µ, Σ.

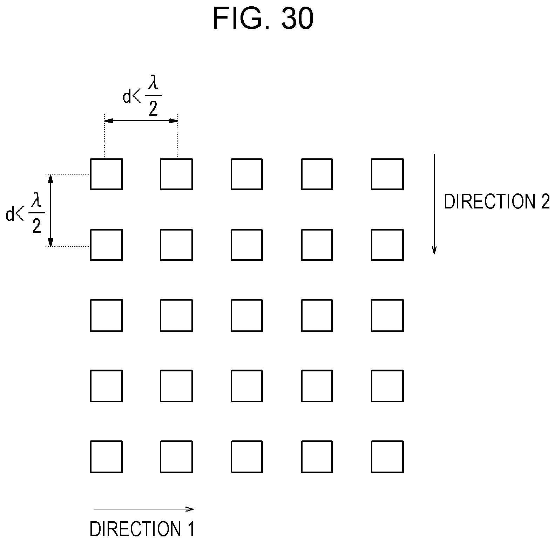

In an embodiment, the transmitting antenna 24 and/or receiving antenna 31 provided in the electronic device 1 is not limited to an arrangement like the one illustrated in FIG. 8. For example, in an embodiment, the receiving antenna 31 provided in the electronic device 1 may have a configuration like the one illustrated in FIG. 30. FIG. 30 illustrates an example of a uniform rectangular array (URA) receiving antenna. A URA receiving antenna like the one illustrated in FIG. 30 may be adopted to perform direction-of-arrival estimation for two angles by the URA receiving antenna alone, without using a beamformer to change the directivity in the transmitting antenna array 24.

In the electronic device 1 illustrated in FIG. 2, the signal processing unit 10 is described as being provided with functional units such as the heartbeat extraction unit 13 and the calculation unit 14. However, in an embodiment, the processing performed by the heartbeat extraction unit 13 and/or the calculation unit 14 may also be performed by an external computer, processor, or the like.

In an embodiment, the processing in step S14 (singular value decomposition/processing to analyze principal components) illustrated in FIG. 12 may be substituted with another subspace method.

In an embodiment, the processing in step S14 (singular value decomposition/processing to analyze principal components) illustrated in FIG. 12 may be omitted if the number of samples N of one chirp can be set to a large number. In an embodiment, the processing in step S14 (singular value decomposition/processing to analyze principal components) illustrated in FIG. 12 may also be omitted if the mixing of other noise can be eliminated by a hardware-based method or the like.

In the processing in step S23 (generate heart sound envelope waveform) illustrated in FIG. 13, the result of the continuous wavelet transform is calculated by summation in the frequency-axis direction according to [Pseudocode 2]. However, the envelope waveform may also be generated by another technique, such as a moving average or the Hilbert transform.

In an embodiment, the electronic device 1 may also calculate the RRI by taking the intervals between negative peaks in the output of the reconstruction error of the autoencoder in the power spectral density (PSD) of the RRI illustrated in FIG. 29. That is, in an embodiment, the electronic device 1 may use the graph in FIG. 29 illustrating the reconstruction error of the autoencoder instead of the heart sound envelope curve illustrated in FIG. 24 to further obtain points corresponding to the RRI as negative peaks in FIG. 29. The time resolution in this case is limited to the moving step of the window in [Pseudocode 3]. A smaller time resolution may be obtained by simply reducing the moving step of the window in [Pseudocode 3].

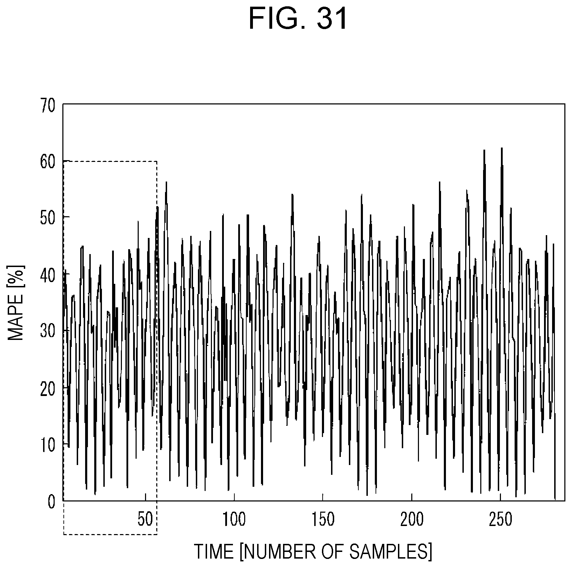

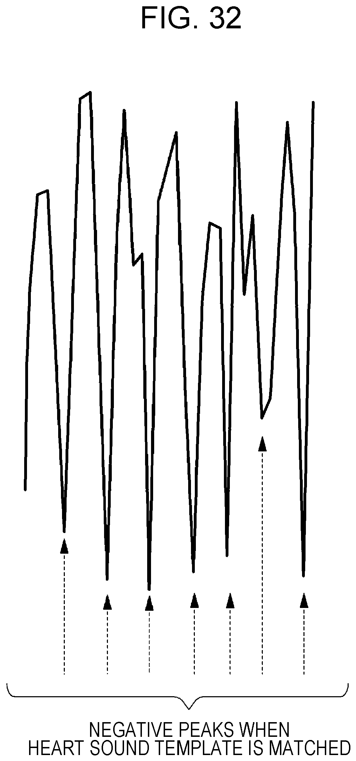

FIGs. 31 and 32 are diagrams for describing negative peaks in the reconstruction error output of an autoencoder. FIG. 31, like FIG. 21(A), illustrates the MAPE of the envelope curve of the best heart sound. The horizontal axis of FIG. 3 represents time in units of the number of samples, and the vertical axis of FIG. 31 represents the MAPE (as a percentage). FIG. 32 illustrates an enlarged view of the portion enclosed by the dashed lines in FIG. 31. As illustrated in FIG. 32, the negative peaks when a heart sound template is matched can be obtained from the graph illustrating the reconstruction error of the autoencoder.

In an embodiment, the electronic device 1 may use the processing in step S25 of FIG. 13 to estimate the Gaussian mixture distribution by a variational Bayesian method. That is, in an embodiment, the electronic device 1 may use the processing in step S25 of FIG. 13 only in place of the histogram of heart sound intervals illustrated in FIGs. 22 and 23. In this case, the Gaussian mixture distribution becomes single-peaked, that is, one Gaussian distribution. In this example, the time intervals between negative peaks in FIGs. 31 and 32 calculated by the method described above may be converted to a histogram and used in the calculation in step S26 illustrated in FIG. 13. The time intervals will correspond to only the heart sound intervals. The histogram in this example may be assumed to have single-envelope characteristics. However, in this example, when several types of noise are included, a variational Bayesian method may be used to find the originally desired times between the negative peaks in FIG. 32 by separation with a Gaussian mixture distribution (of the times between the negative peaks in FIG. 32 and time intervals due to other noise peaks).

In an embodiment, the electronic device 1 may also execute the processing in step S25 of FIG. 13 by Markov chain Monte Carlo methods (MCMC).

As described above, in an embodiment, the electronic device 1 uses, for example, a millimeter-wave sensor provided with a plurality of transmitting antennas and a plurality of receiving antennas to detect weak vibrations such as a heartbeat. In an embodiment, when the subject is not detected, the electronic device 1 detects the body motion of the subject while varying the transmission phase of the antennas to create a beamforming pattern of the transmitting antennas. On the other hand, in an embodiment, once the body motion of the subject is detected, the electronic device 1 carries out beamforming in the direction of the body motion to detect the heartbeat. In this way, according to an embodiment, the electronic device 1 can improve the signal quality by automatically detecting the direction of a human body. Consequently, according to an embodiment, the electronic device 1 can improve the heartbeat detection precision and/or detection range. Thus, according to an embodiment, the electronic device 1 can detect the heartbeat of a human being with high precision.

The present disclosure has been described on the basis of the drawings and examples, but note that a person skilled in the art could easily make various variations or revisions on the basis of the present disclosure. Consequently, it should be understood that these variations or revisions are included in the scope of the present disclosure. For example, the functions and the like included in each functional unit may be rearranged in logically non-contradictory ways. Multiple functional units or the like may be combined into one, or a functional unit may be divided. Each embodiment according to the present disclosure described above is not limited to being carried out exactly according to each embodiment as described, and may be carried out by combining features or omitting some features, as appropriate. In other words, the content of the present disclosure enables a person skilled in the art to make various variations and revisions on the basis of the present disclosure. Therefore, these variations and revisions are included in the scope of the present disclosure. For example, in each embodiment, each functional unit, means, step, and the like can be added to another embodiment or replaced by each functional unit, means, step, and the like of another embodiment in logically non-contradictory ways. In each embodiment, multiple functional units, means, steps, and the like can be combined into one, or each functional unit, means, step, and the like can be divided. Each embodiment according to the present disclosure described above is not limited to being carried out exactly according to each embodiment as described, and can be carried out by combining features or omitting some features, as appropriate.

The embodiments described above are not limited solely to embodiments of the electronic device 1. For example, the embodiments described above may also be carried out as a method of controlling a device like the electronic device 1. Furthermore, the embodiments described above may also be carried out as a program to be executed by a device like the electronic device 1, for example, or as a storage medium or recording medium in which the program is recorded.

In the embodiments described above, the electronic device 1 is described as including components such as the transmitting antenna 24 and the receiving antenna 31 that form what is called a radar sensor. However, in an embodiment, the electronic device may be carried out as a configuration like the signal processing unit 10, for example. In this case, the signal processing unit 10 may be carried out as a unit having functions for processing signals handled by the transmitting antenna 24, the receiving antenna 31, and the like.

### REFERENCE SIGNS

- 1: electronic device
- 10: signal processing unit
- 11: signal generation processing unit
- 12: received signal processing unit
- 13: heartbeat extraction unit
- 14: calculation unit
- 21: transmission DAC
- 22: transmission circuit
- 23: millimeter-wave transmission circuit
- 24: transmitting antenna
- 31: receiving antenna
- 32: mixer
- 33: reception circuit
- 34: reception ADC
- 50: communication interface
- 60: external device

## Claims

1. An electronic device comprising:
a transmission unit configured to transmit a transmission wave;
a reception unit configured to receive a reflected wave of the transmission wave from a target; and
a signal processing unit configured to detect a distance position, direction, and velocity of the target on the basis of a converted signal obtained by taking the Fourier transform of a beat signal of the transmission wave and the reflected wave, wherein
the signal processing unit is provided with an extraction unit configured to extract heartbeat information about the target on the basis of the detected distance, direction, and velocity of the target, and
the extraction unit is configured to
extract signal components corresponding to vibration associated with the heartbeat of the target from the converted signal by using a plurality of window functions,
select heart sound signals that are most appropriate as heart sound by applying a learning classifier to the extracted signal components,
select an optimum signal that is most appropriate as the heartbeat by performing envelope processing on the most appropriate heart sound signals, and
acquire values indicating intervals between peak times from the optimum signal, and extract a heartbeat interval of the target on the basis of the variance and the median of the values indicating intervals.

2. The electronic device according to claim 1, wherein the extraction unit is configured to
extract the signal components by focusing on a plurality of positions in the converted signal, and
execute processing to remove noise from the extracted signal components by using singular value decomposition, the processing using the frequency of the extracted signal components.

3. The electronic device according to claim 1, wherein the extraction unit is configured to perform
processing to extract a micro-Doppler component having a point group where the target is present by applying a plurality of window functions to the converted signal,
processing to perform at least one of principal component analysis and singular value decomposition on the extracted micro-Doppler component,
processing to perform frequency filtering using at least one of the short-time Fourier transform, the continuous wavelet transform, or a bandpass filter on the result of performing at least one of principal component analysis or singular value decomposition, and
multiresolution analysis including a discrete wavelet transform using wavelet and scaling functions appropriate for heart sound on the frequency-filtered result.

4. The electronic device according to claim 1, wherein
the extraction unit is configured to use a learning classifier to determine the optimality, as a heart sound signal, of the signal components corresponding to vibration associated with the heartbeat of the target, and
use moving variance processing to extract an envelope signal from the signal component determined to be optimum by the learning classifier.

5. The electronic device according to claim 1, wherein the extraction unit is configured to
extract envelope signals by using any of the continuous wavelet transform, a discrete wavelet transform, wavelet scattering coefficients, mel-frequency cepstral coefficients, or moving variance processing on the signal components, and
determine an optimum envelope signal on the basis of a score assessed for heart sound of the signal components by using a trained learning classifier on the envelope signals.

6. The electronic device according to claim 5, wherein the extraction unit is configured to
acquire interval values between peak times from the extracted optimum envelope signal and estimates the variance and the median of the value set of the interval values,
create a criterion for extracted the heartbeat interval from the envelope signal on the basis of the estimated variance and median, and
use the criterion to extract the heartbeat interval from the envelope signal.

7. The electronic device according to claim 6, wherein the extraction unit is configured to
detect first heart sound and second heart sound on the basis of the extracted optimum envelope signal, and
assess the heartbeat interval of the target by using DP matching on the basis of a histogram of intervals or a probability density distribution with respect to intervals between the first heart sound and the second heart sound and intervals between the second heart sound and the first heart sound.

8. A method of controlling an electronic device, the method comprising:
transmitting a transmission wave from a transmission unit;
receiving, at a reception unit, a reflected wave of the transmission wave from a target;
detecting a distance position, direction, and velocity of the target on the basis of a converted signal obtained by taking the Fourier transform of a beat signal of the transmission wave and the reflected wave; and
extracting heartbeat information about the target on the basis of the detected distance, direction, and velocity of the target, wherein
the extracting of heartbeat information about the target involves
extracting signal components corresponding to vibration associated with the heartbeat of the target from the converted signal by using a plurality of window functions,
selecting heart sound signals that are most appropriate as heart sound by applying a learning classifier to the extracted signal components,
selecting an optimum signal that is most appropriate as the heartbeat by performing envelope processing on the most appropriate heart sound signals, and
acquiring values indicating intervals between peak times from the optimum signal, and extracting a heartbeat interval of the target on the basis of the variance and the median of the values indicating intervals.

9. A program causing an electronic device to execute a process comprising:
transmitting a transmission wave from a transmission unit;
receiving, at a reception unit, a reflected wave of the transmission wave from a target;
detecting a distance position, direction, and velocity of the target on the basis of a converted signal obtained by taking the Fourier transform of a beat signal of the transmission wave and the reflected wave; and
extracting heartbeat information about the target on the basis of the detected distance, direction, and velocity of the target, wherein
the extracting of heartbeat information about the target involves
extracting signal components corresponding to vibration associated with the heartbeat of the target from the converted signal by using a plurality of window functions,
selecting heart sound signals that are most appropriate as heart sound by applying a learning classifier to the extracted signal components,
selecting an optimum signal that is most appropriate as the heartbeat by performing envelope processing on the most appropriate heart sound signals, and
acquiring values indicating intervals between peak times from the optimum signal, and extracting a heartbeat interval of the target on the basis of the variance and the median of the values indicating intervals.
